(19) Europäisches Patentamt European Patent Office Office européen des brevets

(11) **EP 4 401 741 B1**

(12) **EUROPEAN PATENT SPECIFICATION**

(45) Date of publication and mention of the grant of the patent:
**10.09.2025 Bulletin 2025/37**

(21) Application number: **22801214.2**

(22) Date of filing: **14.09.2022**

(51) International Patent Classification (IPC):
*A61K 31/568* (2006.01)    *A61K 9/00* (2006.01)
*A61P 3/08* (2006.01)    *A61K 38/26* (2006.01)

(52) Cooperative Patent Classification (CPC):
**A61K 31/568; A61K 9/0019; A61K 9/0024; A61P 3/08**

(86) International application number:
**PCT/IB2022/000517**

(87) International publication number:
**WO 2023/041980 (23.03.2023 Gazette 2023/12)**

(54) **BIOACTIVE MOLECULES FOR USE IN TREATING INSULIN RESISTANCE AND/OR RESTORING GLUCOSE HOMEOSTASIS**

BIOAKTIVE MOLEKÜLE ZUR VERWENDUNG BEI DER BEHANDLUNG VON INSULINRESISTENZ UND/ODER WIEDERHERSTELLUNG VON GLUCOSEHOMÖOSTASE

MOLÉCULES BIOACTIVES À UTILISER DANS LE TRAITEMENT DE L'INSULINORÉSISTANCE ET/ OU LA RESTAURATION DE L'HOMÉOSTASIE DU GLUCOSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.09.2021 PCT/FR2021/051573**

(43) Date of publication of application:
**24.07.2024 Bulletin 2024/30**

(73) Proprietor: **PALTECH**
**75007 Paris (FR)**

(72) Inventors:
• **MALBERT, Charles-Henri**
**35170 Bruz (FR)**
• **ALLOUCHE, Maurice Reginald**
**75007 Paris (FR)**

(74) Representative: **Regimbeau**
**20, rue de Chazelles**
**75847 Paris Cedex 17 (FR)**

(56) References cited:
• **MALBERT CHARLES-HENRI ET AL: "Glucose Sensing Mediated by Portal Glucagon-Like Peptide 1 Receptor Is Markedly Impaired in Insulin-Resistant Obese Animals", DIABETES, vol. 70, no. 1, 1 January 2021 (2021-01-01), US, pages 99 - 110, XP055921762, ISSN: 0012-1797, Retrieved from the Internet <URL:https:// watermark.silverchair.com/db200361.pdf? token=AQECAHi208BE49Ooan9kkhW_Er cy7Dm3ZL_9Cf3qfKAc485ysgAAArwwggK4Bgk qhkiG9w0BBwagggKpMllCpQlBADCCAp4GCSq GSlb3DQEHATAeBglghkgBZQMEAS4wEQQMs4 P-MGC7fk8uZ3giAgEQgllCb8zxAkGmEjulwjicTC GCyFQFV2Q-ck982eTJPrR9EgW-lOtLW5YGwI WeXWDGgqJGig6pAFxNwyx5zwusz1zf M6aWkbw> DOI: 10.2337/db20-0361**
• **MALBERT CHARLES-HENRI: "Diabète et obésité : disparition du détecteur du glucose au niveau de l'abdomen", 26 October 2020 (2020-10-26), XP055921769, Retrieved from the Internet <URL:https://www.inrae.fr/sites/default/ files/pdf/CP_Diabetes_V4.pdf> [retrieved on 20220517]**

**(Cont. next page)**

- GUMUSLU ESEN ET AL: "Exenatide upregulates gene expression of glucagon-like peptide-1 receptor and nerve growth factor in streptozotocin/nicotinamide-induced diabetic mice", FUNDAMENTAL & CLINICAL PHARMACOLOGY., vol. 32, no. 2, 20 November 2017 (2017-11-20), FR, pages 174 - 180, XP055922348, ISSN: 0767-3981, Retrieved from the Internet <URL:https://api.wiley.com/onlinelibrary/tdm/v1/articles/10.1111%2Ffcp.12329> DOI: 10.1111/fcp.12329
- ZHU LIYING ET AL: "Glucagon-like peptide-1 receptor expression and its functions are regulated by androgen", BIOMEDICINE & PHARMACOTHERAPY, ELSEVIER, FR, vol. 120, 24 October 2019 (2019-10-24), XP085902713, ISSN: 0753-3322, [retrieved on 20191024], DOI: 10.1016/J.BIOPHA.2019.109555
- VAHL TORSTEN ET AL: "Signaling from GLP-1 receptors in the hepatic portal bed is required for oral glucose tolerance", DIABETES, AMERICAN DIABETES ASSOCIATION, US, vol. 52, no. Suppl.1, 17 June 2003 (2003-06-17), pages A78, XP009168894, ISSN: 0012-1797
- MALBERT CHARLES-HENRI ET AL: "Pancreatic GLP-1r binding potential is reduced in insulin-resistant pigs", BMJ OPEN DIABETES RESEARCH & CARE, vol. 8, no. 2, 1 November 2020 (2020-11-01), pages e001540, XP055921772, Retrieved from the Internet <URL:https://drc.bmj.com/content/bmjdrc/8/2/e001540.full.pdf> DOI: 10.1136/bmjdrc-2020-001540

**Description**

**Field of the invention**

The invention relates to the recovery of glucose-arrival signals arising from a particular portal area (i.e., the portal sensor). The therapeutic goal is to restore the vagal and not vagal afferent signals associated with the post-prandial arrival of glucose in the portal system. More particularly, the invention relates to bioactive molecules and uses thereof in treating insulin resistance and/or restoring glucose homeostasis.

**Background of the invention**

The maintenance of plasma glucose homeostasis is dependent on the integration of glucose-sensing mechanisms located in the brain, portal vein, and intestine (Soty et al., 2017). There is compelling evidence that the portal glucose sensor plays a pivotal role in glucose homeostasis (Berthoud, 2004), (Mithieux, 2020) and vagally-mediated information from the portal sensor is responsible for the regulatory response to portal hyperglycemia including during the post-prandial state (Balkan & Li, 2000), (Vahl et al., 2007). Glucagon-like peptide-1 receptor (GLP-1r) is associated critically with portal glucose sensing (Nishizawa et al., 2013). In particular, GLP-1r are expressed by vagal afferents in the wall of the portal vein (Berthoud & Neuhuber, 2000), GLP-1 infusion was required for portal glucose to stimulate insulin response (Balkan & Li, 2000), co-infusion of glucose and a GLP-1r antagonist into the portal vein inhibits glucose clearance and increases glycemia transiently (Burcelin et al., 2001), and vagal GLP-1r knock-down blunts postprandial insulin release and increases glycemia (Krieger et al., 2016). Collectively, the outcomes of these studies suggest that an impaired capacity of the portal vagal sensor to detect hyperglycemia, as a result of a defective GLP-1r system, is associated with insulin-resistant obesity.

Conversely, several lines of evidence indicate that the GLP-1 system is impaired in insulin-resistant individuals and that GLP-1 resistance may be fundamental to the pathophysiology of prediabetes (Müller et al., 2019). Using PET imaging with a new positron-emitting probe and single-cell electrophysiological methods linked by image-based abdominal navigation, it has been demonstrated, in obese insulin-resistant animals, that the function of the portal vagal glucose sensor is disordered such that portal glucose, within the glucose concentrations likely to occur after a meal, is not detected (Malbert et al., 2020b). This insensitivity of vagal afferents to glucose in obese insulin-resistant, but not lean, animals is a consequence of reduced portal GLP-1r density, especially between the porta hepatis and the left gastric vein, a feature further demonstrated by the suppression of the glucose effect on the vagal afferent by pharmacological inhibition of GLP-1r, in lean animals only (Malbert et al., 2020b).

Vahl T. et al. "Signaling from GLP-1 receptors in the hepatic portal bed is required for oral glucose tolerance", DIABETES vol. 52, no. Suppl.1, 2003 page A78, discloses the importance of portal delivery for GLP-1r associated effects. It discloses that the effect seen in glucose tolerance with a GLP-1 antagonist were only present after administration of the protein to the portal vein and not after administration to the jugular vein.

The change in the distribution of GLP-1r along the portal vein induced by insulin resistance in obesity is important- there is a lower density of receptors between porta hepatic and the left gastric vein in the obese, in contrast to a larger density in lean animals (Malbert, 2021b). A probable consequence of this anatomo-functional arrangement is that the glucose will be detected less in the insulin-resistant obese. This would include venous blood not only from the stomach, but also the superior duodeno-pancreatic vein that branches at the same level as the gastric veins. Deregulation of the peripheral glucose sensors may be important in obesity and type 2 diabetes because peripheral glucose sensors control physiological functions, which when deregulated, trigger mechanisms relevant to the pathogenesis of obesity and diabetes. This cascade has recently been demonstrated in a preclinical model (Malbert et al., 2020b).

Type 2 diabetes (T2D) and obesity are major global health problems and are associated with various other diseases (e.g., cardiovascular disease, obstructive sleep apnea, stroke, peripheral arterial disease, microvascular complications, and osteoarthritis). In 2021, there are 246 million people worldwide with diabetes, and it is estimated that 380 million people will have diabetes by 2025. When T2D cannot be managed with lifestyle changes, it is commonly treated using metformin and/or GLP-1r agonists, which may be taken orally or injected subcutaneously. However, as the population of patients with T2D is heterogeneous, not all respond to such treatments. In addition, these treatments are associated with undesirable side effects such as nausea, vomiting, and diarrhea.

Thus, there remains a need for additional treatments of T2D and related diseases, including insulin resistance and obesity.

**Summary of the invention**

The present invention is defined by the claims.

Any references in the description to methods of treatment refer to the compounds, pharmaceutical compositions and medicaments of the present invention for use in a method for treatment of the human (or animal) body by therapy (or for diagnosis).

The present invention relates to a bioactive molecule upregulating GLP-1r for use in treating insulin resistance and/or restoring glucose homeostasis comprising local administration of said bioactive molecule to the peri-portal region of the portal vein. Indeed, the inventors have found that such administration restores the portal glucose sensor in obese insulin-resistant patients. Restoration of the portal glucose sensor is notably achieved by pharmacologically enhancing the expression of GLP-1r. With local administration, dosage is advantageously reduced. Side effects associated with bioactive molecules are also advantageously reduced. Indeed, gastro-intestinal GLP-1 receptors are suspected to mediate the adverse side-effects of GLP-1 agonists (Jones et al., 2020).

Administration of said bioactive molecule is via a trans-gastric route, a trans-vena cava route, or by direct laparoscopic access, preferably via a trans-gastric route because it can be done as natural orifice surgical access (NOS). Preferably, administration to the peri-portal region of the portal vein is at least partially in the wall of the portal vein and/or in the connective tissue surrounding the portal vein.

Preferably, the bioactive molecule for the use provided herein is a steroid. Preferably, the steroid is androgen, and more preferably is dihydrotestosterone (DHT).

Preferably, the bioactive molecule for the use provided herein is administered at a dose that is at least about 10-fold inferior to the $ED_{50}$ of the molecule, in particular when administered in the peri-portal region, more preferably that is about 100-fold inferior to the $ED_{50}$ of the molecule, in particular when administered in the peri-portal region. More preferably, the bioactive molecule for the use provided herein is administered at a dose that is from about 10-fold inferior to about 100-fold inferior to the $ED_{50}$ of the molecule, in particular when administered in the peri-portal region.

Yet more preferably, the bioactive molecule for the use provided herein is administered at a dose that is at least 10-fold inferior to the $ED_{50}$ of the molecule, in particular when administered in the peri-portal region, more preferably that is 100-fold inferior to the $ED_{50}$ of the molecule, in particular when administered in the peri-portal region. Still yet more preferably, the bioactive molecule for the use provided herein is administered at a dose that is from 10-fold inferior to 100-fold inferior to the $ED_{50}$ of the molecule, in particular when administered in the peri-portal region.

The bioactive molecule for the use provided herein is administered in the peri-portal region, preferably at a dose that is equal or superior to 100 $\mu$g/24h for dihydrotestosterone, more preferably at a dose that is from about 100 $\mu$g/24h to about 1000 $\mu$g/24h. Yet more preferably, the bioactive molecule for the use provided herein is administered, at a dose that is from 100 $\mu$g/24h to 1000 $\mu$g/24h.

Preferably, the bioactive molecule for the use provided herein is administered in a volume that is equal or inferior to 1 mL per 24h period.

Preferably, the bioactive molecule for the use provided herein is administered to a subject that is obese, has type 2 diabetes, has metabolic syndrome, has portal hypertension, pre diabetes, or any combination of two or more thereof.

Preferably, the bioactive molecule for the use provided herein is administered to a subject expressing residual GLP-1r activity in the peri-portal region. In other words, the bioactive molecule for the use provided herein is preferably administered to a subject in which the density of GLP-1r in the peri-portal region is reduced.

Preferably, the reduction in the density of GLP-1r is confirmed via molecular imaging methods, one of which could be positron emission tomography (PET) using a GLP-1r positron emitting ligand, preferably in combination with radio-opaque compound administered IV for further anatomical localization using computed tomography (CT). Both methods within the scope of hybrid imaging of the portal area are suitable for computing an adequate segmentation of the region to emphasize the precise 3D volume in which GLP-1r expression is reduced.

Preferably, an effective amount of the bioactive molecule for the use provided herein is administered by means of a device in the peri-portal region or in the portal vein wall.

## Detailed description

Before describing the present invention in detail, it is to be understood that the invention is not limited to particularly exemplified aspects and may, of course, vary. It is also to be understood that the terminology used herein is for the purpose of describing particular embodiments of the invention only, and is not intended to be limiting. Furthermore, the practice of the invention employs, unless other otherwise indicated, conventional techniques of protein chemistry, molecular biology, and pharmacology, which are within the skill of the art.

In the claims which follow and in the preceding description, the words "comprise," "comprises," "comprising," and other variations are used in an inclusive sense, i.e., to specify the presence of the stated features but not to preclude the presence or addition of further features in various embodiments of the invention, except where the context requires otherwise due to express language or necessary implication. Furthermore, the terms "a," "an," and "the," as used herein include plural forms unless the content of the present application clearly dictates otherwise. As an example, "a bioactive molecule" therefore also includes two or more bioactive molecules.

According to a first aspect, the present invention relates to a bioactive molecule upregulating GLP-1r for use in treating insulin resistance and/or restoring glucose homeostasis by local administration of said bioactive molecule to the peri-portal region of the portal vein.

The term "bioactive molecule upregulating GLP-1r" as used herein refers to any molecule that directly or indirectly leads to an increase in the density of GLP-1 receptors associated with portal glucose sensing. Expression of GLP-1r in the vessel wall of the hepatic portal vein or its vicinity is notably increased. In particular, the expression, and thus the density, of GLP-1r may be increased by modulating the transcription or translational control of the GLP-1R gene coding for GLP-1r or by changes in the trafficking of the receptor itself in particular during the process of its internalization. Increased expression of GLP-1r advantageously at least partially, preferably completely, restores the sensitivity of the portal glucose sensor to portal glucose. Indeed, glucose sensing mediated by GLP-1r is notably impaired in insulin-resistant conditions. In some cases, said bioactive molecule may also increase the activity of the GLP-1r.

Preferably, expression of GLP-1r is increased by at least 10% as compared to the initial level observed in a subject, preferably at least about 20%, 30%, 40%, 50% 60%, 70%, 80%, 90% increase, or even 100% as compared to the initial level observed in a subject. Preferably, expression of GLP-1r is increased by at least 2-fold, 3-fold, or 4-fold, 5-fold, or more. Preferably, expression of GLP-1r is increased to a level similar or identical to that of a healthy subject (i.e., a subject having glucose homeostasis and/or who shows no insulin resistance).

The term "insulin resistance" as used herein refers to a pathological condition of a subject in which the biological responses to the action of insulin in the tissues of said subject are diminished. In other words, insulin resistance is a condition where circulating insulin produces a subnormal biological response. As a non-limiting example, insulin resistance may result from a decreased quantity of insulin receptors in target tissues, e.g., liver, (skeletal) muscle and/or adipose tissues, or from defects in the insulin signaling pathways in cells of such target tissues. Insulin resistance may lead to impaired glucose metabolism and compensatory oversecretion of insulin by the pancreas. Accordingly, the term "insulin resistance" as used herein is also intended to encompass the usual diagnosis of insulin resistance made by any of a number of methods known in the art, including but not limited to: measurement of fasting glucose level, oral or intravenous glucose tolerance test, measurement of fasting insulin level, and the euglycemic glucose clamp test. Impaired fasting glucose (IFG), impaired glucose tolerance (IGT) and/or fasting hyperinsulinemia may serve as diagnostic markers for insulin resistance. IFG or IGT are often referred to as pre-diabetes. Impaired fasting glycemia (IFG) occurs if the glucose concentration 8 hours after the fasting plasma glucose test is between 110 and 125 mg/dl (i.e., between 6.1 and 6.9 mmol/l). Impaired glucose tolerance (IGT) occurs if the glucose concentration after 2 hours of oral glucose tolerance test is between 140 and 199 mg/dl (i.e., between 7.8 to 11.0 mmol/l).

Diseases or disease states in which insulin resistance is commonly present and/or in which insulin resistance is, or is suspected to be, a factor contributing to the etiology of the disease include insulin resistance (a pre diabetes condition when the insulin secretion is preserved), type 2 diabetes, metabolic syndrome, Familial Combined Hyperlipidemia (FCHL), polycystic ovary syndrome (PCOS), familial hypertriglyceridemia, hypercholesterolemia, dyslipidemia, hepatic steatosis, obesity, primary diabetes mellitus (DM), hypertension, heart failure, atherosclerosis, cardiovascular diseases, lipodystrophy (genetic or acquired, e.g., HAART=highly active antiretroviral therapy), fatty liver, inflammation, and Cushing's syndrome.

The term "glucose homeostasis" as used herein refers to promoting the balance of insulin and glucagon in a subject, which helps to maintain blood glucose at the level of a healthy subject. More particularly, glucose homeostasis refers to postprandial glucose homeostasis, in other words, blood glucose levels that are balanced and do not reach extremely high or extremely low glucose levels which are associated with hyperglycemia or hypoglycemia, respectively. Typically, glucose homeostasis in humans refers to blood glucose levels within the range of 70 to 130 mg/dL before meals and no more than 180 mg/dL after meals. In non-diabetic subjects, postprandial glucose usually does not exceed 140 mg/dL and is typically maintained around 100 mg/dL. In contrast, "impaired glucose homeostasis" refers to a transient or persistent condition wherein a subject is unable to maintain normal levels of glucose in the blood.

"Local administration" as used herein refers to a delivery that does not rely upon transport of the bioactive molecule to its intended target site via the vascular, lymphatic, airways or digestive systems. For example, the bioactive molecule may be delivered by injection or implantation of the molecule itself or by injection or implantation of a device containing the molecule. In some cases, local administration occurs in the vicinity of the target site, such that the bioactive molecule diffuses to the intended target site. In the context of the present invention, the bioactive molecule is locally administered to the peri-portal region of the portal vein. The "peri-portal region of the portal vein" refers to the region of the portal vein comprising the portal glucose sensor. The peri-portal region notably comprises the region between the porta hepatic and left gastric veins. It comprises the portal vein itself (i.e., the lumen and walls of the portal vein) as well as the tissues surrounding the portal vein. The peri-portal region of the portal vein notably does not comprise pancreatic or intestinal tissues. Indeed, while GLP-1r is also expressed in these tissues, the bioactive agent is not targeted to them. The specific targeting of the peri-portal region advantageously reduces side effects of the bioactive molecule that may be associated e.g., with administration of the molecule to these tissues. The peri-portal region may be identified by imaging, such as by positron emission tomography (PET) using a GLP-1r positron emitting ligand. As a non-limiting example, the GLP-1r positron emitting ligand comprises a GLP-1r agonist, such as exendin 4, labelled with $_{68}$Ga, $_{18}$F, or $_{64}$Cu

radionuclide. Preferably, the ligand comprises a chelator such as DOTA, tetraazacyclododecane tetraacetic acid or DTPA, diethylenetriaminepentaacetic acid, or DO3A, preferably DO3A. Preferably, the GLP-1r positron emitting ligand is $[_{68}Ga]$ Ga-DO3A-VS-Cys40-Exendin-4 (VS is vinyl sulfone; see e.g., Velkiyan et al., 2017) or 18F-TTCO-Cys40-Exendin-4 (see e.g., Wu et al., 2013), more preferably $[_{68}Ga]$Ga-DO3A-VS-Cys40-Exendin-4. In some cases, the GLP-1r images obtained by PET are further associated with radio-opaque marker enhanced computed tomography (CT). The GLP-1r positron emitting ligand is administered by IV injection.

Preferably, local administration to the peri-portal region of the portal vein is via a trans-gastric route, a trans-vena cava route, or by direct laparoscopic access. The trans-gastric route refers to the accession of the peri-portal region through the wall of a patient's stomach. The trans-vena cava route refers to the accession of the peri-portal region via the superior or inferior vena cava. "Direct laparoscopic access" or "laparoscopic access" as used herein refers to accession via the peritoneal cavity by laparoscopy. In one aspect, the molecule is administered to the interior of the portal vein (i.e., in the lumen), and as such diffuses outwards. In another aspect, the molecule is administered to the tissues surrounding the portal vein, and as such notably diffuses inwards. In yet another aspect, the molecule is administered at least partially, preferably mainly or even completely, in the wall of the portal vein. Preferably, the bioactive molecule is administered to at least partially in the wall of the portal vein and/or in the tissues surrounding the portal vein.

Preferably, the bioactive molecule upregulating GLP-1r is a steroid, and any other suitable agent capable of upregulating the GLP-1r within the area described above. Preferably, the bioactive molecule exhibits GLP-1r upregulating properties in an *in vitro* or in an *in vivo* model. These models include the expression of GLP-1r on pancreatic cells, mesangiome cells, CHO-K1, MIN6B1 but other cells types are equally suitable so far they express GLP-1r. Examples of models that can been used in studies involving activity on GLP-1r of dihydrotestosterone have been reported in Kim, D.-i., et al., (2015), Zhou et al., (2001), Pan et al., (2009), Jones et al., (2018), Gumuslu et al., (2018), Marzook et al., (2021), Zhu et al., (2019). A "steroid" as used herein refers to an organic compound containing in its chemical nucleus the cyclopenta[a] phenanthrene ring system. Steroids upregulating GLP-1r are preferably selected among androgens. Androgens notably include plasterone, stanlorone, testosterone propionate, testosterone enanthate, thiometherone, dehydroepiandrosterone (DHEA), androstenedione, androstenedione, androstenedione, and dihydrotestosterone (DHT). Preferably, the androgen is DHT or a functional variant thereof. Indeed, it has notably been established that androgen steroids can regulate GLP-1r (Zhu et al., 2019). Without being limited by theory, DHT may notably modify transcription of the GLPIR gene as the GLPIR gene promoter region comprises an Ar motif that can be bound by DHT when it is coupled to its receptor (the androgen receptor Ar). The effectiveness of testosterone on insulin sensitivity is in line with the recent data obtained in human but with dose about 10 times larger (Dandona et al., 2021). Furthermore, the reported improvement in glucose metabolism has been demonstrated for example in male after androgen deprivation therapy (Grossmann et al., 2013) while we observed an identical effectiveness of our therapeutical approach irrespective of the sex of the animal. Finally, pre diabetes is associated with increased risk of testosterone deficiency independent of obesity and metabolic syndrome in man (Ho et al., 2013). While the intricacies of the effect of androgens on diabetes is still debatable, it became clear that the action of androgens towards glucose metabolism did not involve their classical pathway after the translocation of the ligand-receptor complex in the cell nucleus to up or down regulate the transcription of relevant genes (Kovacs et al., 1984). Indeed, Navarro and colleagues found that androgen and its receptor could stimulate insulin secretion depending on the activation of the GLP-1R pathway in the cytoplasm in male mouse and human islets (Navarro et al., 2016).

A "functional variant" as used herein refers to a protein that is structurally different from the bioactive molecule but that retains all of the essential functional characteristics of said molecule (i.e., that induces expression of GLP-1r in the same manner). The variant may notably be a naturally-occurring or a non-naturally occurring variant.

Preferably, the bioactive molecule is administered, preferably in the peri-portal region, at a dose that is at least about 10-fold inferior to the $ED_{50}$ of the molecule, and more preferably at a dose that is about 100-fold inferior to the $ED_{50}$ of the molecule. More preferably, the bioactive molecule for the use provided herein is administered, preferably in the peri-portal region, at a dose that is from about 10-fold inferior to about 100-fold inferior to the $ED_{50}$ of the molecule. Yet more preferably, the bioactive molecule for the use provided herein is administered, preferably in the peri-portal region, at a dose that is at least 10-fold inferior to the $ED_{50}$ of the molecule, more preferably that is 100-fold inferior to the $ED_{50}$ of the molecule. Still yet more preferably, the bioactive molecule for the use provided herein is administered, preferably in the peri-portal region, at a dose that is from 10-fold inferior to 100-fold inferior to the $ED_{50}$ of the molecule when administered in the peri-portal region.

The term "about" used here in, for example in expressions such as "a dose that is about x" or "from about xx to about xxx" may be interpreted, if necessary, with regard to the usual uncertainties of drug dosage, and in particular be interpreted as meaning a dose that is of 15% more or less of x, xx or xxx, preferably 10% more or less of x, xx or xxx, more preferably 5% more or less of x, xx or xxx.

The "$ED_{50}$" of the molecule as used herein refers to the median effective dose 50 of the bioactive molecule that produces a therapeutic response or desired effect in 50% of the subjects that are taking it for a particular medical use. The $ED_{50}$ as used herein for DHT corresponds to the $ED_{50}$ of its effect in muscle protein synthesis. The $ED_{50}$ of DHT is thus 20 mg daily (in the form of gel applied cutaneous). Alternatively, remission of diabetes in T2D induced by hypogonadism in male humans was adequately achieved using subcutaneous implants delivering 1000 mg of DHT for 12 weeks e.g. 11 mg/24h (Haider et al., 2020), (Dandona et al., 2021). Therefore, preferably, the bioactive molecule is dihydrotestosterone, and is administered, preferably in the peri-portal region, at a dose that is equal or superior to 100 µg/24h. More preferably, the dihydrotestosterone is administered, preferably in the peri-portal region, at a dose from about 100 µg/24h to about 1000 µg/24h. Yet more preferably, the bioactive molecule for the use provided herein is administered, preferably in the peri-portal region, at a dose that is from 100 µg/24h to 1000 µg/24h.

Preferably, the bioactive molecule is administered in a volume that is equal or inferior to 1 mL per 24h period. Indeed, the volume of the anatomical tissue engulfing the portal sensor dictates also the rate of infusion that cannot be above 1 ml per 24H without a significant leakage to the adjacent abdominal organs (Solass et al., 2016). Preferably, the bioactive molecule is administered by perfusion. In one aspect, the bioactive molecule is administered by means of a device in the portal vein comprising an effective amount of the bioactive molecule.

Preferably, the bioactive molecule administered in a composition which further comprises at least one pharmaceutically acceptable excipient. The term "pharmaceutically acceptable excipient" as used herein refers to a component, or combination of components, that is compatible with the bioactive molecule, does not generate unwanted side-effects in the patient, and that is generally considered to be non-toxic. A pharmaceutically acceptable excipient is most commonly implicated in facilitating administration of the bioactive molecule, increasing product shelf-life or efficacy, or improving the solubility and/or stability of the bioactive molecule. Pharmaceutically acceptable excipients or carriers are well-known in the prior art and can easily be adapted by the skilled person based on the desired galenic formulation of the composition. The galenic formulation and method of local administration (i.e., chosen among trans-gastric, trans-vena cava, and laparoscopic access), and dosage can further be determined based on widely-accepted criteria for adapting patient treatments, including their general health, age, weight, tolerance to treatment, etc., as necessary.

For example, for the trans-gastric route, the excipient is advantageously designed to increase the bioactive molecule residence time at the location of the administration and to limit possible vascular transfer from the peri-portal area.

Yet for example, for the trans-vena cava route, the excipient is advantageously designed to be incorporated in a synthetic matrix so to release slowly, preferably over several months, the bioactive molecule in the peri-portal area.

As another example, for the laparoscopic access, the one or more pharmaceutically acceptable excipients included in the composition of the present invention may further comprise one or more buffers, suspending agents, solubilizing agents, diluents, solvents, preservatives, tonicity adjusting agents, vehicles, and stabilizers. Preferably, the pharmaceutically acceptable excipient comprises at least one tonicity adjusting agent, more preferably sodium chloride at an isotonic concentration. If a vehicle is present, said vehicle is preferably water.

Preferably, the bioactive molecule is administered to a subject having insulin resistance and/or abnormal glucose homeostasis. The subject may notably have any disease or disease state described above that is associated with insulin resistance. Preferably, the bioactive molecule is administered to a subject that is obese, has type 2 diabetes, has metabolic syndrome, has portal hypertension, or any combination of two or more thereof. More preferably, the bioactive molecule is administered to a subject that is obese with insulin resistance. Although it is now clear in the medical field that obesity and insulin resistance are almost always associated, the bioactive molecule as used according to the present invention is shown to be efficient on both clinical features, i.e., body weight, body fat amount repartition, etc., as well as glucose metabolism parameters such as insulin sensitivity.

The term "obesity" as used herein includes all forms of abnormal body weight and weight gain, with distinction that overweight refers to BMI 25-30 kg/m$^2$; obesity refers to BMI within the range of 30 to 35 kg/m$^2$, and morbid obesity refers to BMI higher than 35 kg/m$^2$ according to WHO.

"Type 2 diabetes" refers to a chronic, life-long (i.e., progressing over several years or decades) disease characterized by insulin resistance. Typically, type 2 diabetes is diagnosed when the fasting blood glucose concentration of a subject is above 126 mg/dL (6.4 mmol/L) on two occasions according to the American diabetes association.

"Metabolic syndrome" as used herein refers to a cluster of abnormalities which tend to co-occur in a subject notably involving insulin resistance, obesity, high serum low density lipoprotein (LDL) cholesterol levels, low serum high density lipoprotein (HDL) cholesterol levels, high serum triglyceride levels, and high blood pressure (hypertension). According to the National Cholesterol Education Program of the NIH, diagnosis of metabolic syndrome is made in the presence of any three of the following abnormalities: truncal obesity, defined as waist circumference of more than 102 cm for men and more than 89 cm for women; high serum levels of triglycerides, i.e., 150 mg/dL or higher; low serum levels of HDL cholesterol, i.e., below 40 mg/dL for men and below 50 mg/dL for women; high blood pressure, i.e., 130/85 mm Hg or higher; impaired fasting glucose, i.e., 110 mg/dL or higher. It has been proposed that at least some of these abnormalities may result from an attempt to compensate for insulin resistance.

"Portal hypertension" as used herein refers to a higher pressure in the portal vein and its branches. It is generally defined as a portal pressure gradient (the difference in pressure between the portal vein and the hepatic veins) of at least 5 mm Hg. Although this gradient defines portal hypertension, it should be noted that a gradient of 10 mm Hg or greater defines clinically significant portal hypertension. Preferably, the subject has a portal hypertension of at least 5 mm Hg, of 6-10 mm Hg, or of at least 10 mm Hg.

The terms "subject" and "patient" are used interchangeably herein and refer to a mammal including, but not limited to, a human. The human subject may be an adult or a child of any age. The term "adult" refers more particularly to an individual of at least 18 years of age. A "normal" or "healthy" subject refers to a subject that does not have a particular phenotype, e.g., an insulin resistance phenotype or disease described here (e.g., type 2 diabetes, obesity).

GLP-1r is preferably detected in the peri-portal region of a subject. Indeed, the presence of detectable GLP-1r indicates that the subject remains capable of expressing GLP-1r. Preferably, the density of GLP-1r that is detected in the peri-portal region of a subject is inferior to the density that is detected in a healthy subject. In other words, the density of GLP-1r in the peri-portal region of the subject is reduced as compared to the density of GLP-1r in a healthy subject. Indeed, a reduced density of GLP-1r in a subject is indicative of an abnormal low level of GLP-1r expression in said subject. Such a subject is thus in need of a treatment whereby expression of GLP-1r may potentially be increased, thus increasing in turn the density of GLP-1r in the peri-portal region of the subject.

Preferably, the presence and/or reduction in the density of GLP-1r is confirmed via positron emission tomography (PET) using a GLP-1r positron emitting ligand as described herein. Preferably PET is performed in combination with radio-opaque marker enhanced computed tomography (CT). Preferably, CT together with the PET image is suitable for computing an adequate segmentation of the region with lower GLP-1r expression.

The present invention further relates to a method of treating insulin resistance and/or restoring glucose homeostasis in a subject in need thereof, comprising the steps of:

a) providing at least one bioactive molecule upregulating the expression of GLP-1r, and
b) locally administering said at least one bioactive molecule to the peri-portal region of the portal vein.

The present invention further relates to a method of treating insulin resistance and/or restoring glucose homeostasis in a subject in need thereof, comprising the steps of:

a) providing at least one bioactive molecule upregulating the expression of GLP-1r, and
b) administering said at least one bioactive molecule to the peri-portal region of the portal vein,

wherein the bioactive molecule is administered at a dose that is at least about 10-fold inferior to the $ED_{50}$ of said bioactive molecule, preferably at a dose that is about 100-fold inferior to the $ED_{50}$ of the molecule, and more preferably at a dose that is from about 10-fold inferior to about 100-fold inferior to the $ED_{50}$ of the molecule. Yet more preferably, the bioactive molecule is administered at a dose that is at least 10-fold inferior to the $ED_{50}$ of said bioactive molecule, preferably at a dose that is 100-fold inferior to the $ED_{50}$ of the molecule, even more preferably at a dose that is from 10-fold inferior to 100-fold inferior to the $ED_{50}$ of the molecule.

Preferably, the bioactive molecule for the use provided herein is administered at a dose that is equal or superior to 100 μg/24h for dihydrotestosterone, more preferably at a dose that is from about 100 μg/24h to about 1000 μg/24h. Yet more preferably, the bioactive molecule for the use provided herein is administered at a dose that is from 100 μg/24h to 1000 μg/24h.

The methods described above are preferably for treating insulin resistance in patients suffering from obesity.

The methods described above comprise all aspects as described herein (e.g., as concerns the bioactive molecule, mode of local administration, and treated conditions). The peri-portal region of the portal vein is notably in proximity to the glucose portal sensor involving GLP-1r. The term "treatment" refers to a process by which one or more symptoms of insulin resistance are improved or completely eliminated and/or wherein glucose homeostasis is restored, as described herein.

Successful treatment may be determined by a variety of methods, including e.g., GLP-1r imagery (performed alone or as complement to additional plasma-based measurement such as, and not exclusively, M values from euglycemic clamp studies), measurement of a patient's HbA1c. GLP-1r imagery is preferred as it may provide an earlier indication of the treatment efficacy. Thus, the method provided herein preferably comprises a further step c) of determining treatment efficacy, more preferably by GLP-1r imagery or measurement of the subject's HbA1c, more preferably by GLP-1r imagery, even more preferably by PET and, optionally, CT, as provided herein.

The present invention further relates to a use of the at least one bioactive molecule according to any of the embodiments described herein for the manufacture of a medicament for treating insulin resistance and/or restoring glucose homeostasis in a subject, wherein the at least one bioactive molecule is locally administered to the peri-portal region of the portal vein.

The present invention further relates to the use of the at least one bioactive molecule according to any of the embodiments described herein in treating insulin resistance and/or restoring glucose homeostasis in a subject, wherein the at least one bioactive molecule is locally administered to the peri-portal region of the portal vein. Preferably, such a use if for treating insulin resistance in patients suffering from obesity.

According to a further aspect, the present invention relates to a kit comprising the bioactive molecule as provided herein and, optionally, instructions for providing or administering the molecule described herein to a subject via local administration.

**Figures**

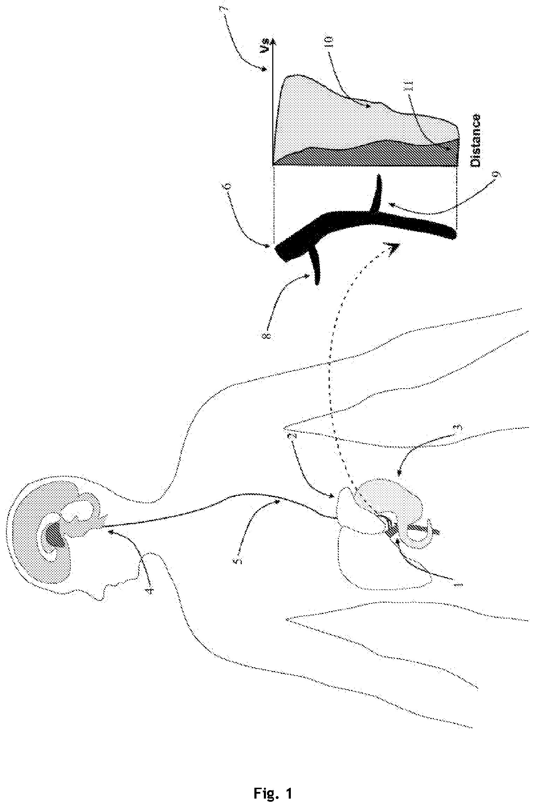

**Figure 1:** Portal glucose sensor and its relation with the vagal afferent for systemic glucose homeostasis. The portal glucose (1) sensor is identifiable through the density of GLP-1r at the vicinity or within the portal vein. It is located at the entrance of the liver (2) and it is partially hidden by the gastroduodenal structure (3). The information arising from the portal glucose sensor is delivered to the dorsal vagal complex (4) via vagal afferents (5). The right lateral insert corresponds to an enlargement of the portal structure (6) at the portal glucose sensor level and the graphs represent the density of GLP1r obtained from molecular imaging of the abdominal area after conversion into quantitative binding potential units (7). The gastric vein together with the duodena-pancreatic vein is indicated as (8) while the splenic vein is indicated as (9). The binding potential histogram indicated as (10) depicted a typical distribution found in lean individual while the one indicated as (11) showed the effect of insulin-resistance obesity.

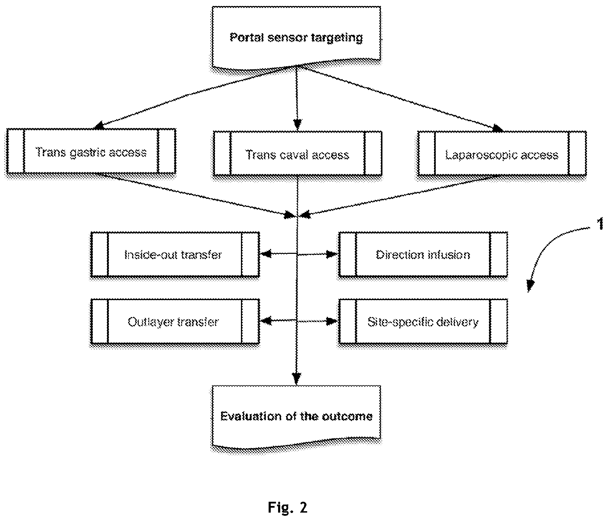

**Figure 2:** Three targeting solutions are proposed, which may notably depend on the presence of any additional pathologies in a patient. The trans-gastric access is particularly preferred as it is the least invasive. Irrespective of the targeting solution selected, several options for the delivery of the bioactive molecule are available (1). Due to the position of the GLP-1r with respect of the portal vein wall, it is possible to administer the bioactive molecule dedicated to up-regulate the GLP-1r either from the inside or the outside of the portal vein itself. The bioactive molecule can be administered with a liquid excipient or in a more complex form with the help of site-specific delivery excipient for extended duration delivery. Finally, the effectiveness of the procedure can be evaluated alongside the duration of the therapy without having to wait for a significant improvement of the classical markers of type 2 diabetes.

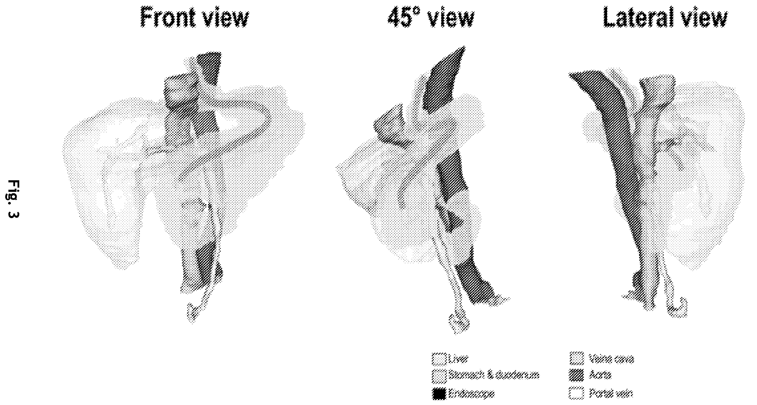

**Figure 3:** Actual tridimensional representation of a possible path for accessing the portal glucose sensor using the trans-gastric route with the help of an endoscope and a steerable probe. The tridimensional shapes of the organs and vessels are calculated from segmentation of an actual contrast enhanced CT exam. The stomach and duodenal wall are made partially transparent to allow the visualization of the virtual endoscope. This represents the first step for a trans-gastric access to the portal sensor.

**Figure 4:** Alternate access point for the trans-gastric route of portal sensor access. This route is advantageous as it may be less demanding for the endoscopist. An echo-endoscope is available for catheter guidance. A - Descending Aorta, B posterior vena cava, C mesenteric artery, D portal vein.

**Figure 5:** Experimental protocol for the preclinical model of insulin resistance

**Figure 6:** Evolution of body weight of animals after DHT infusion or implantation relative to control.

**Figure 7:** Body composition in animals after DHT implantation (squares), compared to control (circles), measured from CT analysis.

**Figure 8:** Daily food intake in animals after DHT implantation or DHT infusion - each dot corresponds the mean of all animals in a group.

**Figure 9:** Example of GLP-1r increased expression after DHT implant insertion at 1-month post-surgery compared to sham implantation.

**Figure 10**: Changes in distribution volume of GLP-1r along the portal vein one month after DHT implant or DHT continuous infusion. Each graph represents the same animal before (circles) and after the surgery (squares).

**Examples**

**1. Patient selection and bioactive molecule**

*Patients selection:* patients with a residual GLP-1r on the portal vein are preferably selected, as they are more likely to respond positively than those having no detectable GLP-1r expression. Likewise, patients with GLP-1r expression on the portal vein at levels comparable to that of a heathy subject may be less likely to respond positively to treatment, as GLP-1r expression may be considered to be normal prior to treatment.

While in normal individuals the portal vein could be easily identified, it is more difficult to locate the boundaries of the structure. Therefore, the first step could be the segmentation of the GLP-1r image on the basis of the injected CT. However, because of the requirement for an attenuation correction of the raw PET image, a prior CT without radio-opaque injection is performed either before or after the PET imaging itself. This un-injected CT is used only for transmission error removal during the PET image reconstruction as performed classically.

*Bioactive molecule dihydrotestosterone (DHT):* the demonstration of an interaction between the pulsatility of testosterone secretion and circulating GLP-1 is recent (Izzi-Engbeaya et al., 2020) but it is now established based on *in vivo* experiments in C57BL/6 and db/db mice that the density of the GLP-1 receptor is regulated by androgens (Zhu et al. 2019). The proposed mechanism of action is based on the partial identity of the DHT/Ar motifs and that of the GLP1r gene. Thus, extracellular DHT diffuses into the cytoplasm and then couples to its receptor (androgen receptor Ar) which can then induce translocation of DHT/Ar to the nuclear level. The DHT/Ar in the nucleus binds to the Ar motif present on the promoter region of the GLP1r gene and increases its transcription.

**2. Imaging associated with administering the bioactive molecule**

Imaging of the GLP-1r-dependent portal sensor is done primarily using positron emission tomography (PET) imaging of GLP-1 receptor (GLP-1r) after the administration of a positron emitting ligand such as for example $[_{68}$Ga]Ga-DO3A-VS-Cys40-Exendin-4. Depending on the condition of the patient, the GLP-1r images can be associated with a radio-opaque marker enhanced computed tomography (CT) suitable for computing an adequate segmentation of the region with lower GLP-1r expression.

2.1 Radioligand synthesis and quality control

Synthesis and quality control procedure for $[_{68}$Ga]Ga-DO3A-VS-Cys40-Exendin-4 are provided here as an example. In labelling with $_{68}$Ga, the requirement for low mass may lead to formation of $_{68}$Ga colloids which, if not properly removed, can be seen as uptake in the spleen and liver (Brom et al., 2016). Thus, a specific quality control procedure is used, as described below. Synthesis of the radiolabeled ligand is done for example using the following procedure. $_{68}$Ga is produced daily by automatic elution of a generator with 0.6M HCL as part of the synthesis process. Production of $[_{68}$Ga]Ga-DO3A-VS-Cys40-Exendin-4 is achieved using a $_{68}$Ga-peptide synthesis cassette with 47 $\mu$g or less of the DO3A-VS-Cys40-Exendin-4 cold molecule dissolved in 2.5 ml Hepes buffer inserted in the reactor. The generic peptide synthesizer sequence is modified to adjust for the temperature (less to 90°C) and heating duration (from 10 to 15 min) of the reactor.

Prior to injection, the final compound is checked for the amount of $_{68}$Ga that was not associated with the receptor ligand and the concentration of Exendin-4 labeled by $_{68}$Ga in solution, using high-performance chromatography comprising a reverse phase C18 column and a UV detector at 220 nm in series with a radiation flow detector. The gradient conditions are as follows: solvent A 10 mM trifluoroacetic acid; solvent B 70% acetonitrile, 30% $H_2O$, and 10 mM trifluoroacetic acid with UV detection at 220 nm; gradient elution is done for 0-2 min at 35% B, 2-9 min at 35%-100% B, and 9-12 min at 100% solvent B; and the flow rate is 2.0 mL/min. In this context, labelled Ga-DO3A-VS-Cys40-Exendin-4 is eluted with a retention time of ~5.1 min. Alternative gradient/analytical methods involving other reverse phase column and different gradient values (yet primarily with Water/acetonitrile) could be done.

2.2 Imaging acquisition and primary processing

Abdominal images are preferably acquired in dynamic PET mode with the field of view comprising the Porta Hepatis and the abdominal aorta, with the latter that could be used for construction of the arterial input function in the absence of an arterial input function obtained from the direct radioactivity measurement of the blood circulating in an extemporaneously constructed arterio-veinous loop according to Malbert et al (2020b). For instance, the dynamic frame sequence could be for 60 min (30 frames; 12×10s, 6×30s, 5×20s, 5×300 s, or 2×600 s). The dynamic sequence is corrected for attenuation using a CT image acquired immediately before or after the radiolabel injection with imaging parameters as required by the scanner manufacturer. Upon completion of the PET imaging, a second, coregistered, CT imaging sequence may be acquired after the injection of a radio-opaque injection solution. The parameters for imaging and injection are similar to those classically achieved for liver first-pass imaging.

Serial veinous sampling can be performed along with image acquisition. Blood sampling can be performed every 20 minutes, but reliable arterial input function requires a minimum of 4 to 5 samples during the 60 min image acquisition. Radioactivity of the plasma is calculated after centrifugation of each blood sample. Radiolabelled compound is administered rapidly at about 0.2 MBq/Kg IV.

Composite arterial function is calculated for each individual preferably using the radioactivity of the arterial blood circulating in an extemporaneously built arterio-veinous loop according to Malbert et al (2020b). However, since this method is relatively invasive and requires the insertion of an arterial catheter, the arterial input function could be approximated using the changes in radio activity measured within a volume of interest centered on the abdominal aorta. Radioactivity of the corresponding plasma mass is calculated using the whole blood to plasma ratio obtained for the discrete blood sample. The measured data are fitted preferably using an iterative algorithm to a 3 exponential curve to achieve a monotonic function. Alternatively, only a single venous sample is obtained after the injection of the radiolabel and the aortic radioactivity can be fitted to a population-based template constructed as the mean of several individuals according to Zanotti-Fregonara et al., 2011.

## 3. Methods of local administration of the bioactive molecule to the peri-portal region of the portal vein

Local administration to the peri-portal region of the portal vein may be performed by several routes of administration, and more particularly via the trans-gastric route, the trans-vena cava route, or by direct laparoscopic access.

### 3.1 Trans-gastric Route

The trans-gastric access to the portal target is the primary route of interest since it is reproducible without excessive patient or physician burden - a mandatory constraint for the application of the invention provided herein.

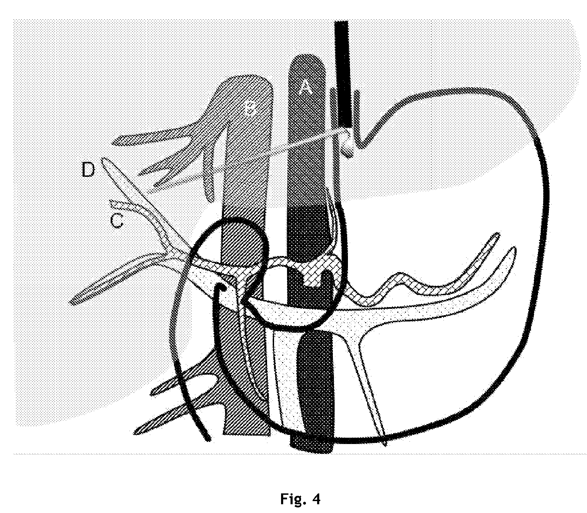

Access to the portal vein outer wall is achieved via a minute opening of the stomach wall, achieved using an endoscope fitted with a catheter. The endoscope suitable for such a procedure can be either a classical multichannel gastroscope or an echo-endoscope for enhanced visualization of the portal vein. As a non-limiting example, several access points are suitable to reach the target area of the portal vein and are illustrated in Figure 4. While the primary location for injection of the bioactive molecule is the peri-portal area, it is also possible to insert, via the same route and after portal puncture, a device designed to infuse the therapeutic molecule from within the portal intima.

CT imaging performed and processed as described in catheter targeting embodiment is uploaded to the tracking computer that comprises adequate interfaces for optical tracking such as polaris vicra infrared camera (NDI, Canada), magnetic tracking such as aurora antenna (NDI) coupled with the mandatory electronic interface for the magnetic transceiver glued on the targeting catheter and a solution to grab the 2D image generated by an echography apparatus. The CT/PET model of the portal sensor registered with the CT images is also uploaded on the targeting computer. Once spatially referenced by pointing both on the computer screen the CT representation of the fiducial markers that are also identified on the patient body, the computer is able to track all optical based tools, namely the echography probe and the position of the magnetic antenna tracking system, in real time thanks to the build-in models for both the probe and the antenna. Similarly, it is possible to display the live ultrasound image with the accurate position of the tip of the catheter superimposed on the 3D live CT projections.

The procedure initially comprises the co-registration steps as indicated above followed by the insertion of the endoscope towards the planned intra-gastric insertion point. The steering capabilities of the endoscope are used to apply the tip of the catheter with the needle retracted onto the gastric mucosa while have a visualization of the system in 3D based on the CT image and the target model. Once in position, the needle is extended and the mobile part of the catheter is inserted through the gastric wall. Immediately after crossing the serosa boundary, the needle is retracted again to avoid further injuries on the close by anatomical structures. The echography probe is used for accurate on-line visualization of the portal system during the additional progression of the inner catheter so that the tip is placed at the immediate vicinity of the portal sensor. The steering capabilities of the catheter tip are designed to help this positioning and to optimize the application of the therapeutic molecule so that as much as possible of the portal sensor is in close vicinity to it. Completion of the procedure includes the retraction of the inner catheter, then the removal of the outer catheter and confirmation of the absence of large gastric wall opening and extraction of the endoscope from the patient.

### 3.2 Trans-vena cava Route

The portal sensor is also accessible via either the superior or the inferior vena cava. While both the external part and the internal part of the portal sensor can be accessed with this method, internal delivery through an end-vascular medical device is preferred since it can be incorporated as part of a routine procedure used to alleviate portal vein stenosis or portal vein hypertension.

Portal vein stents may notably be implanted by either of the following two procedures: ultrasonography-guided percutaneous transhepatic puncture of the intrahepatic portal vein and an insertion via the ileocolic vein. Placement of the stent by either route has been already published in a broader context (Intagliata, Caldwell, & Tripodi, 2019), (Kato et al., 2017).

### 3.3 Laparoscopic access

Direct peri-portal space access may be performed by laparoscopy (this route is also referred to herein as "laparoscopic access") on a patient for which accessibility or other interfering pathologies prevent an endoscopic or radio-guided procedure for the delivery of the bioactive molecule. Patients having, for example, pathologies with important coagulation disorders resulting in possible massive blood loss, should notably undergo laparoscopic access.

Portal sensor location may be pinpointed using at least one laparoscopic capable tool with the necessary 3D optical localizer similar to that described in Malbert, 2021a. It is probable that a certified surgeon will not require the additional electromagnetic tracking and the associated ultrasound visualization.

Specifically, the peri-portal space is accessed close to the portal sensor after a small incision of the peritoneal fascia which is preferably, but not exclusively, done using a ultrasonic scalpel. After blunted dissection of the tissue to access the external layer of the portal vein, a catheter can be inserted alongside the portal vein and the peritoneal fascia is closed using a surgical stitch. The catheter is also anchored at some distance from its entrance to the small cavity of the peritoneum. Alternatively, the bioactive molecule in an appropriate form can be placed 'en lieu' of the catheter alongside the portal vein. In the embodiment using a catheter, its free end is issued under the skin and connected to a device suitable for sterile access through the skin that can dispense the bioactive molecule.

**4. Verification of treatment efficacy**

GLP-1r imagery can be used to assess the success of the portal GLP-1r restoration therapy, with this procedure being repeatable. This image set could be used alone or as a complement to additional plasma-based measurement such as, and not exclusively, M values from euglycemic clamp studies (Beneschet al., 2015), dynamic insulin sensitivity from IVGTT (Pillonetto et al, 2010) or HbA1c. GLP- 1r image is likely to be used as an early indicator of the effectiveness of the therapy unlike HbA1c, for example if compared with reference to a GLP-1r PET that is performed immediately before local administration of the bioactive molecule. This verification is likely to be performed numerous times throughout a patient's lifetime. Therefore, it is designed to be as little invasive as possible and to use a minimal amount of radioactive compound, even if this is detrimental for image quality. In particular, the CT scan - the most irradiating part of the imaging session, can be skipped and an original, pre injection, CT scan can be used after adequate registration using the liver mass as co-reference identifiable both on actual PET and original CT image.

**5. Effectiveness of the method on preclinical model of insulin resistance**

The experimental protocol was designed to evaluate the following:

- Capacity of dihydrotestosterone (DHT) administered at the ED50 dosage to enhance GLP-1r expression at the level of the periportal area
- Capacity of DHT administered at the 1/100 of ED50 dosage to enhance GLP-1r expression at the level of the periportal area
- Capacity of DHT administered at the ED50 dosage to improve insulin sensitivity in comparison with untreated obese animals.
- Capacity of DHT administered at the 1/100 ED50 dosage to improve insulin sensitivity in comparison with untreated obese animals.
- Changes in glucose metabolism parameters induced by DHT administered at 1/100 ED50 dosage in comparison with untreated obese animals.
- Changes in food intake and body weight induced by DHT administered either at ED50 and at 1/100 ED50 dosage in comparison with untreated obese animals.

5.1. Animals and experimental protocol

The method was evaluated on 15 miniature pigs aged 5 years old (INRAE, UEPR, Saint-Gilles, France) and rendered insulin resistant after 3-4 months high fat high sucrose diet ingestion in excess according to already published data (Malbert et al., 2017b). The diet supplied 4024 kcal per kg of feed and was presented twice daily so that the animals could eat up to 150% of the recommended caloric intake (288 kcal/kg $BW^{0.75}$).

Once obese and insulin resistant, the animals were distributed into three groups of equal size (n = 5 animals). One group served as control and was submitted only to a dummy implantation procedure including the initial GLP-1r PET imaging sequence using [$_{68}$Ga]Ga-DO3A-VS-Cys40-Exendin-4. The second group was implanted during a laparoscopic procedure with a silicon catheter (2 mm OD) with its tip located at the portal sensor close to the minimum GLP-1r expression area according to the procedure described in 3.3. The terminal part of the catheter was bend at 90° to enter the periportal area. The catheter was secured at the entrance of the periportal cavity using a dacron surgical mesh (Biomesh P1, Cousin Bioserv Paris) glued to the silicon rubber prior to the implantation. During the implantation, the surgical mesh was glued to the outer layer of the portal vein using a surgical glue dispenser Glubran 2 and Glutack Cousin Bioserv Paris). The opposite tip of the catheter exited between the shoulder of the animal and was connected to an elastomeric pump. The pump (Easypump, II, BBraun) delivered 2mL per hour of a solution of dihydrotestosterone (Sigma, Paris) in sterile water/DMSO (100:5 V/V) so that 10 mg of dihydrotestosterone was infused daily. The amount of the active molecule was irrespective of the body weight (BW). The pump was located in a lateral pocket of a jacket Whose size was adjusted for obese Yucantan minipigs (Lomir, Canada) and wore by the animal for the duration of the experiment. The third group was implanted during a laparoscopic procedure with an implant delivering 100 μg of dihydrostestoterone daily (Belma Technologies, Belgium) irrespective of the weight of the animal. The implant was inserted in the periportal area and the insertion hole achieved on the mesenteric layer for insertion was closed by a patch of surgical mesh glued directly on the meso using a procedure similar that described above. The anesthesia and surgical approach used for laparoscopy were identical those already described by Malbert et al. (Malbert et al., 2017b).

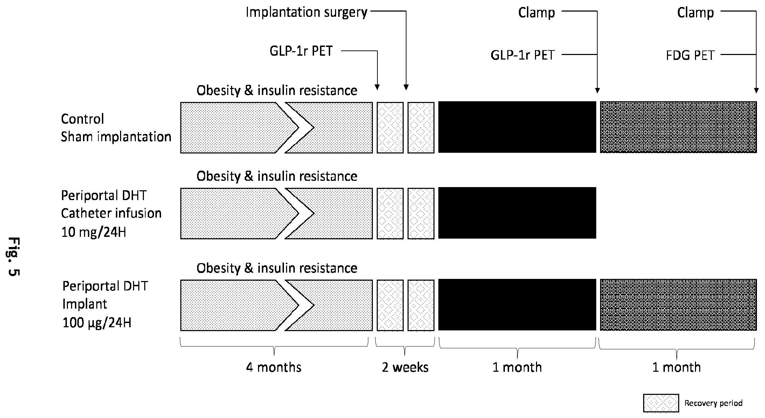

The protocol was described in figure 5 and consisted of two periods; one during which an obese insulin resistant status is achieved followed by a one-month post-surgery period during which DHT was slowly release according to the method of delivery described above. For control and implant groups only, the experimental period was also extended for an additional month to evaluate the capacity of the method to achieve a sustained effect on glucose metabolism. This extended period was not achievable on catheter implanted animals due to ethical constraints. A one-week recovery period is inserted in the protocol after the initial PET GLP-1r scan and after the surgery. The data acquired during these periods were discarded from further analysis.

5.2. Measurements

Three weeks before the first PET imaging session, irrespective of the experimental group, the animals were transferred to the experimental facility and housed individually in a 2,5 m$^2$ pen comprising a robotic feeder that allow to supply two meals daily automatically while monitoring the feeding behavior (Malbert, 2021). The analysis of the data recorded by the feeders are identical those described in Malbert et al. (Malbert et al., 2017a).

The animals were weighted weekly in fasted conditions (e.g. before the morning meal) during the entire experimental period. To avoid alterations of feeding behavior unrelated to treatments, the amount of diet supplied daily was fixed at the beginning of the experimental period irrespective of the future changes in body weight.

Distribution of GLP-1r along the portal vein and within the abdominal organs was obtained during an initial PET scan after the administration of 25 to 40 MBq [68Ga]Ga-DO3A-VS-Cys40-Exendin-4 according to the above section 3 of the examples. The portal area of low GLP-1r expression was identified and used for insertion of either the catheter tip or the DHT implant according to the above section 3.3 relating to laparoscopic access. The anesthesia and overall procedure for imaging is identical that described by Malbert et al (Malbert et al., 2020b). The same PET scan procedure was repeated one month after the surgical procedure allowing to compare between groups and within subjects of the same group the changes in GLP-1r distribution. During the same imaging, as part of the procedure, CT based attenuation was used also for calculation of the body fat, visceral fat and muscle proportion according to Val-Laillet et al (Val-Laillet et al., 2010). Furthermore, the amount of fat present in the liver was estimated using the liver to spleen ratio method (Kodama et al., 2007). Furthermore, during the same anesthesia, blood sample was collected for glycemia measurement, insulinemia and for evaluation of the blood pressure and related parameters using high definition oscillometrie (Egner, 2015).

Glucose metabolism was measured at the whole-body and regional levels using [18]FluoroDeoxyglucose (FDG) PET imaging and euglycemic clamp according to Malbert et al (Malbert et al., 2017b). Briefly, the regional metabolic rate of glucose (MRglu) was calculated using Patlak model free quantitative assessment using PET dynamic images and externally measured arterial input fraction of FDG. The measurements were achieved using PMod software. For these calculations, the values of lump constant were 0,45 for the brain (Poulsen et al., 1997); 1 for liver (Iozzo et al., 2007); 1,2 for striated muscle (Peltoniemi et al., 2000); 1 for heart and pancreas and 1,15 for the intestine. The endogenous glucose production in fasting condition was obtained by extrapolation at infinite of the FDG concentration within the arterial blood after FDG injection according to Iozzo et al (Iozzo et al., 2006). Glucose oxidation rate was obtained using indirect calorimetry obtained before FDG or radioactive exenatide injection while the animal is already anesthetized according to Malbert et al (Malbert et al., 2020a).

Insulin sensitivity was obtained during the plateau of an euglycemic hyperinsulinemic clamp according to the following formula:

$$IS = \frac{GIRss}{Gss \,.\, \Delta Iss}$$

IS - insulin sensitivity (dL/Kg.min/$\mu$U/mL*1E$^{-3}$)
GIRss - glucose infusion rate at clamp plateau (mg/kg.min)
Gss - Plasma glycemia at clamp plateau (mg/dL)
$\Delta$Iss - Difference between basal insulinemia and insulinemia at clamp plateau ($\mu$U/mL)

5.3 Results

### 5.3.1. Phenotypic parameters

The evolution of phenotypic characteristics of mini-pigs in the control, DHT infusion and DHT implant groups was presented in Table 1 for the first 30 days after implantation surgery.

Table 1

|  | Control | DHT infusion 10 mg/24H | DHT implant 100 $\mu$g/24H |
|---|---|---|---|
| Body weight (kg) | 85,3 $\pm$ 2,1 | 80,8 $\pm$ 2,0* | 80,4 $\pm$ 2,7* |
| Fat (% of BW) | 38,1 $\pm$ 1,9 | 36,8 $\pm$ 0,5 | 37,3 $\pm$ 0,7 |
| Abdominal fat (% of BW) | 40,6 $\pm$ 0,9 | 38,1 $\pm$ 0,7! | 38,4 $\pm$ 0,3! |
| Muscle (% of BW) | 6,2 $\pm$ 1,1 | 6,5 $\pm$ 1,0 | 6,5 $\pm$ 0,9 |
| Liver to spleen ratio | 0,93 $\pm$ 0,03 | 1,1 $\pm$ 0,02* | 0,99 $\pm$ 0,02* |
| Daily ingestion (g/kg.day) | 9,9 $\pm$ 0,03 | 8,6 $\pm$ 0,03* | 8,6 $\pm$ 0,03* |
| Mean arterial pressure (mmHg) | 81 $\pm$ 2,4 | 79 $\pm$ 3,1 | 74 $\pm$ 3,2* |
| Systolic blood pressure (mmHg) | 54 $\pm$ 1,5 | 55 $\pm$ 2,1 | 55 $\pm$ 1,6 |
| Pulse rate (min-1) | 111 $\pm$ 3,6 | 104 $\pm$ 8,7 | 102 $\pm$ 3,3* |
| Mean $\pm$ SD. * indicates a significant difference p<0,01 from control. ! indicates a significant difference at 0, 05. N= 5 for all groups. | | | |

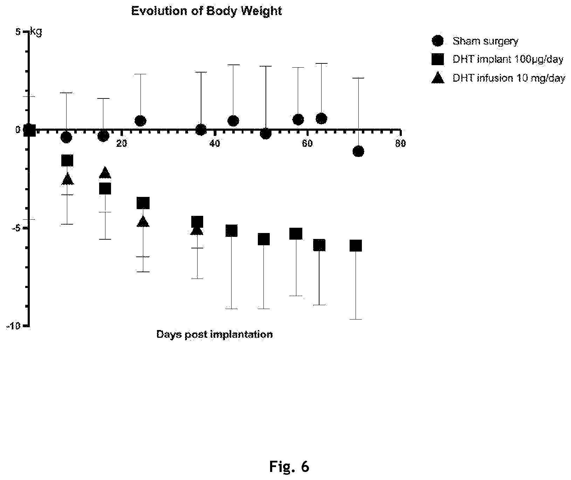

Evolution of body weight relative to control group was depicted in figure 6. Briefly, DHT infusion and DHT implant were equally effective to reduce significantly body weight and the temporal evolution of this reduction was similar between treated groups with a stabilization of the weight about 40 days after the onset of the procedure. Note that due to experimental restriction, the evolution in body weight for the DHT infusion group is unknown after the first 30 days.

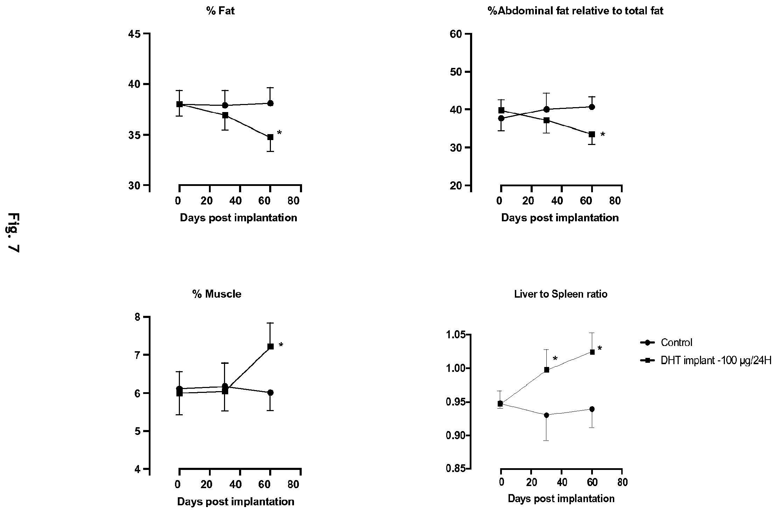

The reduction in body weight was primarily the consequence of a reduction in fat which also is less relative to body weight. This difference became significant at 60 days after implantation (see figure 7). For all treated groups, abdominal fat was significantly reduced while the amount of skeletal muscle is increased especially 60 days after DHT implantation for the implant group. Furthermore, the amount of intrahepatic fat is also reduced in treated groups as indicated by a significant increase in liver to spleen ratio. Note that this increase is even more obvious after 60 days on treatment (see figure 7).

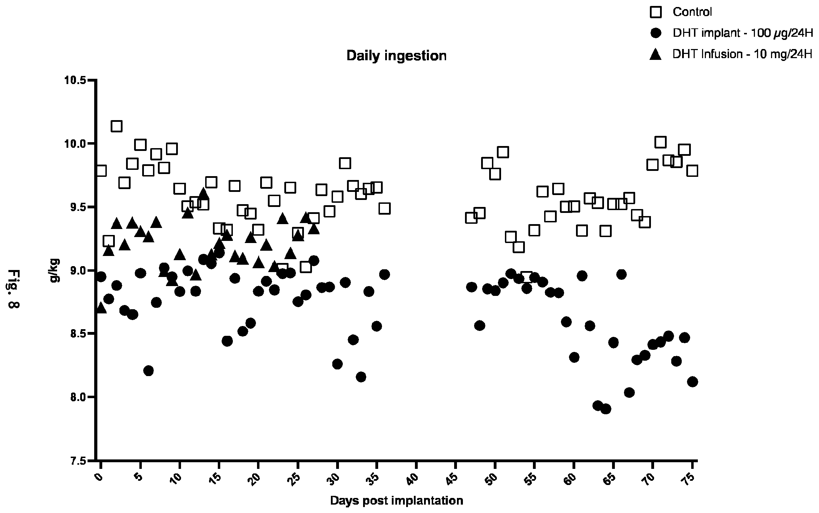

Daily ingestion was significantly reduced in DHT infusion and implant groups. This reduction was observed immediately after the completion of the recovery phase and was maintained at the same lower level during the entire experimental period (see figure 8 for a typical example in DHT implant and infusion groups). The decrease in food intake level was primarily the consequence of a reduced size of the afternoon meal while the morning meal remained unchanged. The behavioral pattern of ingestion was unchanged. The duration of non-ingestion periods and the rate of ingestion were similar between groups but the termination of the afternoon meal was set to an earlier time point resulting ultimately in less diet consumption.

Mean blood pressure was significantly reduced in DHT implant group but not in DHT infusion group compared to sham surgery group. A close feature is also found for heart rate with a significant reduction for the DHT implant group only. Systolic blood pressure remained unchanged by the treatment and this was also true for the DHT implant group 60 days after surgery (56 $\pm$ 2,4 vs 54 $\pm$ 4,2 for DHT implant vs Control group respectively, p>0,05).

### 5.3.2. GLP-1r expression

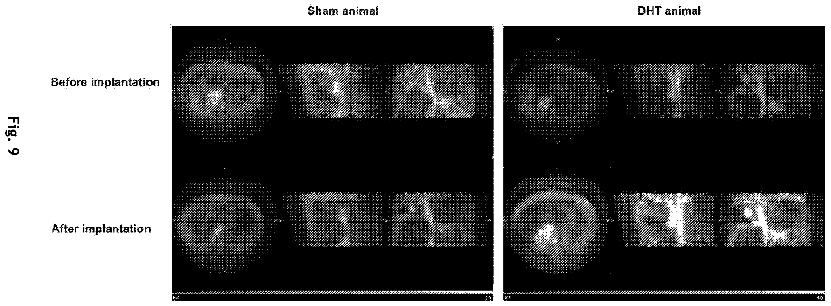

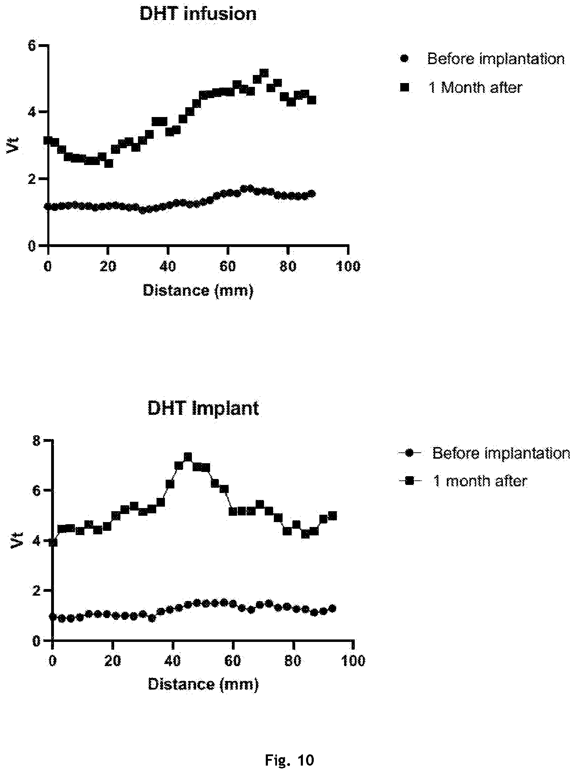

Binding potential/volume of distribution of the portal GLP-1r was more than doubled in DHT infusion and implant groups compared to control (Vt - 3,7 $\pm$ 0,13; 5,2 $\pm$ 0,15 and 1,1 $\pm$ 0,05 mL/ccm for DHT infusion, DHT implant and control group respectively, both DHT groups with p<0,01 compared to control). For example, the detection of the GLP-1r expression after DHT implantation (1-month post-surgery) is depicted in figure 9. This increase was noticed from the porta hepaticus down to the mesenteric branches of the portal vein. However, the increased expression in GLP-1r was substantial downwards of the insertion point of either the catheter or the implant (e.g. between 20 to 50 mm). The overall increase in Vt differed between treated groups; the implant group showing a clearly defined peak at the implant location while the pattern is increasing steadily along the portal vein for the DHT infusion group. Such pattern, suggest some diffusion of DHT in the periportal space at distance of the catheter tip itself (see figure 10).

Significant GLP-1r binding was measured in the abdominal cavity aside the periportal area. This non portal binding reflects pancreatic GLP-1r expression exclusively which was slightly increased in DHT implant but not in DHT infusion group (5,7 ± 0,48; 4,8 ± 0,41 and 4,6 ± 0,61 for DHT implant, DHT infusion and control groups respectively, p<0,05 between DHT implant and control group, ns between DHT infusion vs control group). This observation is important since in diabetic pigs there is a reduced pancreatic GLP-1r expression (Malbert et al., 2020a). The present data demonstrated that impaired expression is reversed by DHT implant and they are supporting identical reduced expression in T2D humans (Eriksson et al., 2022).

### 5.3.3. Glucose metabolism

The key parameters of glucose metabolism obtained 30 days after the implantation surgery in mini-pigs in the control, DHT infusion and DHT implant group were presented in table 2.

**Table 2**

|  | Control | DHT infusion 10 mg/24H | DHT implant 100 μg/24H |
|---|---|---|---|
| Fasting plasma glucose (mmol/L) | 5,3 ± 0,24 | 4,2 ± 0,23* | 4,2 ± 0,27* |
| Fasting plasma insulin (μU/mL) | 6,5 ± 0,31 | 2,1 ± 0,21* | 2,5 ± 0,10* |
| Clamp plasma insulin (μU/mL) | 766 ± 13,8 | 501 ± 14,1* | 380 ± 8,0* |
| Mean plasma glucose at clamp plateau (mmol/L) | 5,7 ± 0,36 | 4,2 ± 0,36* | 4,2 ± 0,28* |
| Insulin sensitivity (dL/kg.min/μMU/mL*1E-3) | 4,4 ± 0,37 | 6,8 ± 0,20* | 6,6 ± 0,28* |
| Basal metabolic rate (kcal/min.kg) | 0,0071 ±0,0005 | 0,0079±0,0007 | 0,0087±0,0007* |
| Fasting glucose oxidation rate (mg/min.kg) | 2,2 ± 0,49 | 1,8 ± 0,10 | 1,8 ± 0,08 |
| Mean ± SD. * indicates a significant difference p<0, 05 from control. N= 5 for all groups. | | | |

Importantly fasting glycemia was significantly less in DHT treated animals compared to controls. The reduced plasma glucose was also associated with reduced circulating insulin which was 1/3 of the insulinemia measured in control group. Measurement of whole-body insulin sensitivity by the gold standard e.g. euglycemic hyperinsulinemic clamp demonstrated a significant one third increase irrespective of the DHT group compared to control. This increased insulin sensitivity was comparable that observed in otherwise identical yet lean miniature pigs (Malbert et al., 2017b).

Indirect calorimetry shows a small but significant increase in basal metabolic rate for DHT implant group only compared to control. This increase is likely the consequence of the larger muscle % in DHT implant but not in DHT infusion group since basal metabolic rate changes are paralleled those in fat free mass (Jéquier, 1989). No significant changes were observed in glucose oxidation, a steadiness that could reflect the condition of the measurement - fasting instead of after a glucose load (Felber et al., 1981).

Regional glucose uptake in the fasting condition were presented for control and DHT implant groups only 2 months after the implantation surgery in table 3.

**Table 3**

|  | Control | DHT implant 100 μg/24H |
|---|---|---|
| Brain glucose uptake (mmol.min$^{-1}$.100g$^{-1}$) | 23 ± 3,5 | 15 ± 4,6* |
| Hepatic glucose uptake (mmol.min$^{-1}$.100g$^{-1}$) | 4,4 ± 1,2 | 5,7 ±2,0* |
| Skeletal muscle glucose uptake (mmol.min$^{-1}$.100g$^{-1}$) | 1,3 ± 0,39 | 5,1 ± 0,11* |
| Heart glucose uptake (mmol.min$^{-1}$.100g$^{-1}$) | 4,1 ± 0,80 | 8,3 ± 0,83* |
| Pancreatic glucose uptake (mmol.min$^{-1}$.100g$^{-1}$) | 1,6 ± 0,8 | 4,1 ± 0,1* |
| Intestinal glucose uptake (mmol.min$^{-1}$.100g$^{-1}$) | 2,1 ± 0,5 | 1,9 ± 0,9 |
| Endogenous glucose production (μmol.min$^{-1}$) | 1493 ± 25,8 | 946 ± 31,4* |
| Mean ± SD. * indicates a significant difference p<0, 05from control. N= 5 for all groups. | | |

In accordance with already published data in humans (Iozzo, 2015) or animals (Malbert, 2021) comparing lean and obese insulin-resistant subjects, all major organs controlling glucose metabolism showed a significant increase in glucose uptake after DHT implant. The increase in glucose uptake is consistent with a beneficial effect as presented in humans after bariatric surgery (Dadson et al., 2016) is ranging between 30 to more than 50% compared to control. No significant difference was measured in intestinal glucose metabolism. Such absence of difference between group is in line with an identical lack of effect, in fasting condition only, observed in obese versus lean individuals (Mäkinen et al., 2015). Finally, brain glucose uptake showed an opposite behavior with a large significant decrease in uptake for the DHT implant group compared to control. Again, this behavior is reminiscent of the enhanced brain glucose uptake associated with insulin resistance in humans (Rebelos et al., 2021), meaning that restoration of insulin sensitivity results likely from a decrease brain glucose uptake. Therefore, the changes induced by DHT implant in glucose uptake parameters, irrespective of the organ under scrutiny are equally consistent with an improvement in glucose metabolism and a restoration of the insulin sensitivity.

Fasting endogenous glucose production was about halved in DHT implant group compared to control. This evolution is identical yet more intense that observed in obese humans after bariatric surgery (Camastra et al., 2011).

**References**

Balkan, B., & Li, X. (2000). Portal GLP-1 administration in rats augments the insulin response to glucose via neuronal mechanisms. American Journal of Physiology-Regulatory, Integrative and Comparative Physiology, 279(4), R1449-R1454.

Benesch et al., (2015). How to Assess the Quality of Glucose Clamps? Evaluation of Clamps Performed With ClampArt, a Novel Automated Clamp Device. Journal of Diabetes Science and Technology, 9(4), 792-800.

Berthoud, H.-R., & Neuhuber, W. L. (2000). Functional and chemical anatomy of the afferent vagal system. Autonomic Neuroscience, 85(1-3), 1-17.

Berthoud, H. R. (2004). Anatomy and function of sensory hepatic nerves. Anat Rec A Discov Mol Cell Evol Biol, 280(1), 827-835.

Brom et al., (2016). The effect of purification of Ga-68-labeled exendin on in vivo distribution. EJNMMI Res, 6(1), 65.

Burcelin et al., (2001). Glucose competence of the hepatoportal vein sensor requires the presence of an activated glucagon-like peptide-1 receptor. Diabetes, 50(8), 1720-1728.

Camastra, S., et al., (2011). Early and longer term effects of gastric bypass surgery on tissue-specific insulin sensitivity and beta cell function in morbidly obese patients with and without type 2 diabetes. Diabetologia, 54(8), 2093-2102

Dadson, P., et al., (2016). Effect of Bariatric Surgery on Adipose Tissue Glucose Metabolism in Different Depots in Patients With or Without Type 2 Diabetes. Diabetes Care, 39(2), 292-299

Dandona, P., et al., (2021). Mechanisms underlying the metabolic actions of testosterone in humans: A narrative review. Diabetes Obes Metab, 23(1), 18-28

Egner, B. (2015). High Definition Oscillometry: Non-invasive Blood Pressure Measurement and Pulse Wave Analysis. Handb Exp Pharmacol, 229, 243-264.

Eriksson, O., et al., (2022). Glucagonlike Peptide-1 Receptor Imaging in Individuals with Type 2 Diabetes. J Nucl Med, 63(5), 794-800.

Eriksson et al., (2017). Species differences in pancreatic binding of DO3A-VS-Cys40 - Exendin4. Acta Diabetol, 54(11), 1039-1045.

Felber, J.-P., et al., (1981). Glucose storage and oxidation in different degrees of human obesity measured by continuous indirect calorimetry. Diabetologia, 20(1), 39-44.

Grossmann, M., et al., (2013). Androgens and prostate cancer; pathogenesis and deprivation therapy. Best Pract Res Clin Endocrinol Metab, 27(4), 603-616.

Haider, K. S., et al., (2020). Remission of type 2 diabetes following long-term treatment with injectable testosterone undecanoate in patients with hypogonadism and type 2 diabetes: 11-year data from a real-world registry study. Diabetes Obes Metab, 22(11), 2055-2068.

Ho, C. H., et al., (2013). Prediabetes is associated with an increased risk of testosterone deficiency, independent of obesity and metabolic syndrome. PLoS One, 8(9), e74173.

Intagliata et al., (2019). Diagnosis, Development, and Treatment of Portal Vein Thrombosis in Patients With and Without Cirrhosis. Gastroenterology, 156(6), 1582-1599.e1.

Iozzo, P., et al., (2007). Quantification of liver glucose metabolism by positron emission tomography: validation study in pigs. Gastroenterology, 132(2), 531-542.

Iozzo, P. (2015). Metabolic imaging in obesity: underlying mechanisms and consequences in the whole body. Annals of the New York Academy of Sciences, 1353(1), 21-40.

Iozzo, P., et al., (2006). 18F-FDG assessment of glucose disposal and production rates during fasting and insulin stimulation: a validation study. Journal of nuclear medicine, 47(6), 1016-1022.

Izzi-Engbeaya et al., (2020). Effects of Glucagon-like Peptide-1 on the Reproductive Axis in Healthy Men. J Clin Endocrinol Metab 105:dgaa072.

Jéquier, E. (1989). Energy metabolism in human obesity. Sozial-und Präventivmedizin, 34(2), 58-62.

Jones et al., (2018). Targeting GLP-1 receptor trafficking to improve agonist efficacy. Nat Commun 9:1602.

Kato, A., et al., (2017). Portal vein stent placement for the treatment of postoperative portal vein stenosis: long-term success and factor associated with stent failure. BMC Surg, 17(1), 11.

Kodama, Y., et al., (2007). Comparison of CT methods for determining the fat content of the liver. AJR Am J Roentgenol, 188(5), 1307-1312

Kovacs, W. J., et al., (1984). A mutation that causes lability of the androgen receptor under conditions that normally promote transformation to the DNA-binding state. J Clin Invest, 73(4), 1095-1104.

Krieger et al., (2016). Knockdown of GLP-1 Receptors in Vagal Afferents Affects Normal Food Intake and Glycemia. Diabetes, 65(1), 34-43.

Mäkinen, J., et al., (2015). Obesity-associated intestinal insulin resistance is ameliorated after bariatric surgery. Diabetologia, 58(5), 1055-1062.

Malbert, C. H., et al., (2017a). Effects of chronic abdominal vagal stimulation of small-diameter neurons on brain metabolism and food intake. Brain Stimul, 10(4), 735-743.

Malbert, C.-H., et al., (2017b). Obesity-associated alterations in glucose metabolism are reversed by chronic bilateral stimulation of the abdominal vagus nerve. Diabetes, 66(4), 848-857.

Malbert et al., (2020a). Pancreatic GLP-1r binding potential is reduced in insulin-resistant pigs. BMJ Open Diabetes Res Care, 8(2).

Malbert et al., (2020b). Glucose-Sensing Mediated by Portal GLP-1 Receptor is Markedly Impaired in Insulin-Resistant Obese Animals. Diabetes.

Malbert, C.-H. (2021a). Open-source 3D printable frameless stereotaxic system for young and adult pigs. Journal of Neuroscience Methods, 359, 109222.

Malbert, C.-H. (2021b). Vagally Mediated Gut-Brain Relationships in Appetite Control-Insights from Porcine Studies. Nutrients, 13(2), 467-484.

Marzook et al., (2021). The Interplay of Glucagon-Like Peptide-1 Receptor Trafficking and Signalling in Pancreatic Beta Cells. Front Endocrinol (Lausanne) 12:678055.

Mithieux, G. (2020). Détection du glucose sanguin par le système nerveux: pourquoi, où, comment.

Miiller et al., (2019). Glucagon-like peptide 1 (GLP-1). Mol Metab, 30, 72-130.

Navarro, G., et al., (2016). Extranuclear Actions of the Androgen Receptor Enhance Glucose-Stimulated Insulin Secretion in the Male. Cell Metab, 23(5), 837-851.

Nishizawa et al., (2013). Intraportal GLP-1 stimulates insulin secretion predominantly through the hepatoportal-pancreatic vagal reflex pathways. Am J Physiol Endocrinol Metab, 305(3), E376-87.

Peltoniemi, P., et al., (2000). Lumped constant for [(18)F]fluorodeoxyglucose in skeletal muscles of obese and nonobese humans. American Journal of Physiology- Endocrinology And Metabolism, 279(5), E1122-30.

Pillonetto et al., (2010). Dynamic insulin sensitivity index: importance in diabetes. Am J Physiol Endocrinol Metab, 298(3), E440-8.

Poulsen, P. H., et al., (1997). In vivo estimation of cerebral blood flow, oxygen consumption and glucose metabolism in the pig by [15O]water injection, [15O]oxygen inhalation and dual injections of [18F]fluorodeoxyglucose. Journal of Neuroscience Methods, 77(2), 199-209.

Rebelos, E., et al., (2021). Insulin Resistance Is Associated With Enhanced Brain Glucose Uptake During Euglycemic Hyperinsulinemia: A Large-Scale PET Cohort. Diabetes Care, 44(3), 788-794.

Solass et al., (2016). Morphology of the peritoneal cavity and pathophysiological consequences. Pleura and peritoneum, 1(4), 193-201.

Soty et al., (2017). Gut-Brain Glucose Signaling in Energy Homeostasis. Cell Metab, 25(6), 1231-1242.

Vahl et al., (2007). Glucagon-Like Peptide-1 (GLP-1) Receptors Expressed on Nerve Terminals in the Portal Vein Mediate the Effects of Endogenous GLP-1 on Glucose Tolerance in Rats. Endocrinology, 148(10), 4965-4973.

Val-Laillet, et al., (2010). A computed tomography scan application to evaluate adiposity in a minipig model of human obesity. Br J Nutr, 104(11), 1719-1728.

Wu et al., (2013)., Development and Evaluation of 18F-TTCO-Cys40-Exendin-4: A PET Probe for Imaging Transplanted Islets. Journal of Nuclear Medicine, 54(2): 244-251.

Zanotti-Fregonara, et al., (2011). Image-derived input function for human brain using high resolution PET imaging with [C](R)-rolipram and [C]PBR28. PLoS One, 6(2), e17056.

Zhu et al., (2019). Glucagon-like peptide-1 receptor expression and its functions are regulated by androgen. Biomedicine & Pharmacotherapy, 120, 109555.

**Claims**

1. Bioactive molecule upregulating GLP-1r for use in treating insulin resistance and/or restoring glucose homeostasis by local administration to the peri-portal region of the portal vein.

2. Bioactive molecule upregulating GLP-1r for the use of claim 1, wherein administration is via a trans-gastric route, a trans-vena cava route, or by direct laparoscopic access, preferably via a trans-gastric route.

3. Bioactive molecule upregulating GLP-1r for the use of claim 1 wherein administration to the peri-portal region of the portal vein is at least partially in the wall of the portal vein and/or in the connective tissue surrounding the portal vein.

4. Bioactive molecule upregulating GLP-1r for the use of any one of claims 1 to 3, wherein the bioactive molecule is a steroid, preferably dihydrotestosterone.

5. Bioactive molecule upregulating GLP-1r for the use of any one of claims 1 to 4, wherein the bioactive molecule is administered at a dose that is at least about 10-fold inferior to the $ED_{50}$ of the molecule, preferably at a dose that is about 100-fold inferior to the $ED_{50}$ of the molecule, more preferably at a dose that is from about 10-fold inferior to about 100-fold inferior to the $ED_{50}$ of the molecule, yet more preferably at a dose that is from 10-fold inferior to 100-fold inferior to the $ED_{50}$ of the molecule.

6. Bioactive molecule upregulating GLP-1r for the use of any one of claims 1 to 5, wherein the bioactive molecule is administered at a dose that is equal or superior to 100 $\mu$g/24h for dihydrotestosterone, more preferably at a dose that is from about 100 $\mu$g/24h to about 1000 $\mu$g/24h, yet more preferably at a dose that is from 100 $\mu$g/24h to 1000 $\mu$g/24h.

7. Bioactive molecule upregulating GLP-1r for the use of any one of claims 1 to 6, wherein the bioactive molecule is administered in a volume that is equal or inferior to 1 mL per 24h period.

8. Bioactive molecule upregulating GLP-1r for the use of any one of claims 1 to 7, the bioactive molecule is administered to a subject that is obese, has type 2 diabetes, has metabolic syndrome, has portal hypertension, pre diabetes, or any combination of two or more thereof.

9. Bioactive molecule upregulating GLP-1r for the use of any one of claims 1 to 8, wherein GLP-1r is detected in the peri-portal region of a subject.

10. Bioactive molecule upregulating GLP-1r for the use of any one of claims 1 to 9, wherein a reduction of the density of GLP-1r in the peri-portal region of a subject as compared to the density of GLP-1r in a healthy subject is detected.

11. Bioactive molecule upregulating GLP-1r for the use of claim 9 or 10, wherein the presence and/or reduction in the density of GLP-1r is determined via positron emission tomography (PET) using a GLP-1r positron emitting ligand, preferably in combination with radio-opaque marker enhanced computed tomography (CT).

12. Bioactive molecule upregulating GLP-1r for the use of any one of claims 1 to 11, wherein the bioactive molecule is administered by means of a device in the portal vein comprising an effective amount of the bioactive molecule.

13. Bioactive molecule upregulating GLP-1r for use in treating insulin resistance and/or restoring glucose homeostasis, wherein said bioactive molecule is administered in the peri-portal region, at a dose that is at least about 10-fold inferior to the $ED_{50}$ of said bioactive molecule, preferably at a dose that is from 10-fold inferior to 100-fold inferior to the $ED_{50}$ of the molecule.

14. Bioactive molecule upregulating GLP-1r for use of claim 13 in treating insulin resistance and/or restoring glucose homeostasis, wherein the bioactive molecule is administered in the peri-portal region, at a dose that is equal or superior to 100 $\mu$g/24h for dihydrotestosterone, preferably at a dose that is from about 100 $\mu$g/24h to about 1000 $\mu$g/24h, more preferably at a dose that is from 100 $\mu$g/24h to 1000 $\mu$g/24h.

**Patentansprüche**

1. Bioaktives Molekül, das GLP-1r hochreguliert, zur Verwendung bei der Behandlung von Insulinresistenz und/oder der Wiederherstellung der Glukosehomöostase durch lokale Verabreichung an die Periportalregion der Pfortader.

2.  Bioaktives Molekül, das GLP-1r hochreguliert, zur Verwendung nach Anspruch 1, wobei die Verabreichung über einen transgastrischen Weg, einen Weg durch die Vena cava oder durch einen direkten laparoskopischen Zugang, vorzugsweise über einen transagastrischen Weg, erfolgt.

3.  Bioaktives Molekül, das GLP-1r hochreguliert, für die Verwendung nach Anspruch 1, wobei die Verabreichung an die Periportalregion der Pfortader zumindest teilweise in der Wand der Pfortader und/oder in dem die Pfortader umgebenden Bindegewebe erfolgt.

4.  Bioaktives Molekül, das GLP-1r hochreguliert, zur Verwendung nach einem der Ansprüche 1 bis 3, wobei das bioaktive Molekül ein Steroid, vorzugsweise Dihydrotestosteron, ist.

5.  Bioaktives Molekül, das GLP-1r hochreguliert, zur Verwendung nach einem der Ansprüche 1 bis 4, wobei das bioaktive Molekül in einer Dosis verabreicht wird, die mindestens etwa 10-mal niedriger ist als die $ED_{50}$ des Moleküls, vorzugsweise in einer Dosis, die etwa 100-mal niedriger als die $ED_{50}$ des Moleküls ist, noch bevorzugter in einer Dosis, die von etwa 10-mal niedriger bis etwa 100-mal niedriger als die $ED_{50}$ des Moleküls ist, und noch bevorzugter in einer Dosis, die von 10-mal niedriger bis 100-mal niedriger als die $ED_{50}$ des Moleküls ist.

6.  Bioaktives Molekül, das GLP-1r hochreguliert, für die Verwendung nach einem der Ansprüche 1 bis 5, wobei das bioaktive Molekül in einer Dosis verabreicht wird, die gleich oder höher als 100 μg/24h für Dihydrotestosteron ist, noch bevorzugter in einer Dosis, die von etwa 100 μg/24h bis etwa 1000 μg/24h beträgt, und noch bevorzugter in einer Dosis, die von 100 μg/24h bis 1000 μg/24h beträgt.

7.  Bioaktives Molekül, das GLP-1r hochreguliert, zur Verwendung nach einem der Ansprüche 1 bis 6, wobei das bioaktive Molekül in einem Volumen verabreicht wird, das gleich oder weniger als 1 ml pro 24-Stunden-Zeitraum beträgt.

8.  Bioaktives Molekül, das GLP-1r hochreguliert, zur Verwendung nach einem der Ansprüche 1 bis 7, wobei das bioaktive Molekül einem Patienten verabreicht wird, der adipös ist, an Typ-2-Diabetes leidet, ein metabolisches Syndrom hat, an portaler Hypertonie leidet, an Prä-Diabetes erkrankt ist oder eine beliebige Kombination von zwei oder mehreren davon aufweist.

9.  Bioaktives Molekül, das GLP-1r hochreguliert, zur Verwendung nach einem der Ansprüche 1 bis 8, wobei GLP-1r in der Periportalregion eines Patienten nachgewiesen wird.

10. Bioaktives Molekül, das GLP-1r hochreguliert, zur Verwendung nach einem der Ansprüche 1 bis 9, wobei eine Verringerung der Dichte von GLP-1r in der Periportalregion eines Patienten im Vergleich zur Dichte von GLP-1r bei einem gesunden Patienten nachgewiesen wird.

11. Bioaktives Molekül, das GLP-1r hochreguliert, für die Verwendung nach Anspruch 9 oder 10, wobei das Vorhandensein und/oder die Verringerung der Dichte von GLP-1r mittels Positronen-Emissions-Tomographie (PET) unter Verwendung eines GLP-1r-Positronen emittierenden Liganden, vorzugsweise in Kombination mit einer durch radio-opake Marker verstärkten Computertomographie (CT), bestimmt wird.

12. Bioaktives Molekül, das GLP-1r hochreguliert, zur Verwendung nach einem der Ansprüche 1 bis 11, wobei das bioaktive Molekül mittels einer Vorrichtung in die Pfortader verabreicht wird, die wirksame Menge des bioaktiven Moleküls enthält.

13. Bioaktives Molekül, das GLP-1r hochreguliert, zur Verwendung bei der Behandlung von Insulinresistenz und/oder der Wiederherstellung der Glukosehomöostase, wobei das bioaktive Molekül in der Periportalregion in einer Dosis verabreicht wird, die mindestens etwa 10-mal niedriger als die $ED_{50}$ des bioaktiven Moleküls ist, vorzugsweise in einer Dosis, die von 10-mal niedriger bis 100-mal niedriger als die $ED_{50}$ des Moleküls ist.

14. Bioaktives Molekül, das GLP-1r hochreguliert, für die Verwendung nach Anspruch 13 bei der Behandlung von Insulinresistenz und/oder der Wiederherstellung der Glukosehomöostase, wobei das bioaktive Molekül in der Periportalregion in einer Dosis verabreicht wird, die gleich oder höher als 100 μg/24h für Dihydrotestosteron ist, vorzugsweise in einer Dosis, die von etwa 100 μg/24h bis etwa 1000 μg/24h beträgt, noch bevorzugter in einer Dosis,

die von 100 μg/24h bis 1000 μg/24h beträgt.

**Revendications**

1. Molécule bioactive régulant à la hausse le GLP-1r destinée à être utilisée dans le traitement de l'insulinorésistance et/ou la restauration de l'homéostasie du glucose par administration locale dans la région périportale de la veine porte.

2. Molécule bioactive régulant à la hausse le GLP-1r pour l'utilisation selon la revendication 1, dans laquelle l'administration se fait par voie transgastrique, par voie transveineuse cave ou par accès laparoscopique direct, de préférence par voie transgastrique.

3. Molécule bioactive régulant à la hausse le GLP-1r pour l'utilisation selon la revendication 1, dans laquelle l'administration dans la région périportale de la veine porte se fait au moins partiellement dans la paroi de la veine porte et/ou dans le tissu conjonctif entourant la veine porte.

4. Molécule bioactive régulant à la hausse le GLP-1r pour l'utilisation selon l'une quelconque des revendications 1 à 3, dans laquelle la molécule bioactive est un stéroïde, de préférence la dihydrotestostérone.

5. Molécule bioactive régulant à la hausse le GLP-1r pour l'utilisation selon l'une quelconque des revendications 1 à 4, dans laquelle la molécule bioactive est administrée à une dose qui est au moins environ 10 fois inférieure à la $DE_{50}$ de la molécule, de préférence à une dose qui est environ 100 fois inférieure à la $DE_{50}$ de la molécule, plus préférablement à une dose qui est d'environ 10 fois inférieure à environ 100 fois inférieure à la $DE_{50}$ de la molécule, plus préférablement encore à une dose qui est de 10 fois inférieure à 100 fois inférieure à la $DE_{50}$ de la molécule.

6. Molécule bioactive régulant à la hausse le GLP-1r pour l'utilisation selon l'une quelconque des revendications 1 à 5, dans laquelle la molécule bioactive est administrée à une dose égale ou supérieure à 100 μg/24 h pour la dihydrotestostérone, plus préférablement à une dose d'environ 100 μg/24 h à environ 1 000 μg/24 h, plus préférablement encore à une dose comprise qui est de 100 μg/24 h à 1 000 μg/24 h.

7. Molécule bioactive régulant à la hausse le GLP-1r pour l'utilisation selon l'une quelconque des revendications 1 à 6, dans laquelle la molécule bioactive est administrée dans un volume qui est égal ou inférieur à 1 ml par période de 24 h.

8. Molécule bioactive régulant à la hausse le GLP-1r pour l'utilisation selon l'une quelconque des revendications 1 à 7, la molécule bioactive étant administrée à un sujet obèse, atteint de diabète de type 2, du syndrome métabolique, d'hypertension portale, de prédiabète, ou de toute combinaison de deux ou plusieurs de ceux-ci.

9. Molécule bioactive régulant à la hausse le GLP-1r pour l'utilisation selon l'une quelconque des revendications 1 à 8, dans laquelle le GLP-1r est détecté dans la région périportale d'un sujet.

10. Molécule bioactive régulant à la hausse le GLP-1r pour l'utilisation selon l'une quelconque des revendications 1 à 9, dans laquelle une réduction de la densité du GLP-1r dans la région périportale d'un sujet par rapport à la densité du GLP-1r chez un sujet sain est détectée.

11. Molécule bioactive régulant à la hausse le GLP-1r pour l'utilisation selon la revendication 9 ou 10, dans laquelle la présence et/ou la réduction de la densité du GLP-1r est déterminée par tomographie par émission de positons (TEP) à l'aide d'un ligand émettant des positons GLP-1r, de préférence en combinaison avec une tomodensitométrie (TDM) améliorée par un marqueur radio-opaque.

12. Molécule bioactive régulant à la hausse le GLP-1r pour l'utilisation selon l'une quelconque des revendications 1 à 11, dans laquelle la molécule bioactive est administrée dans la veine porte au moyen d'un dispositif comprenant une quantité efficace de la molécule bioactive.

13. Molécule bioactive régulant à la hausse le GLP-1r destinée à être utilisée dans le traitement de l'insulinorésistance et/ou la restauration de l'homéostasie du glucose, dans lequel ladite molécule bioactive est administrée dans la région périportale, à une dose qui est au moins environ 10 fois inférieure à la $DE_{50}$ de ladite molécule bioactive, de préférence à une dose qui est de 10 fois à 100 fois inférieure à la $DE_{50}$ de la molécule.

**14.** Molécule bioactive régulant à la hausse le GLP-1r destinée à être utilisée selon la revendication 13 dans le traitement de la résistance à l'insuline et/ou la restauration de l'homéostasie du glucose, dans lequel la molécule bioactive est administrée dans la région périportale, à une dose qui est égale ou supérieure à 100 $\mu$g/24 h pour la dihydrotestostérone, de préférence à une dose qui est d'environ 100 $\mu$g/24 h à environ 1 000 $\mu$g/24 h, plus préférablement à une dose qui est de 100 $\mu$g/24 h à 1 000 $\mu$g/24 h.

EP 4 401 741 B1

Fig. 1

Fig. 2

Fig. 3

Fig. 4

Fig. 5

Fig. 6

Fig. 7

Fig. 8

Fig. 9

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VAHL T. et al.** Signaling from GLP-1 receptors in the hepatic portal bed is required for oral glucose tolerance. *DIABETES*, 2003, vol. 52, A78
- **BALKAN, B.** ; **LI, X.** Portal GLP-1 administration in rats augments the insulin response to glucose via neuronal mechanisms. *American Journal of Physiology-Regulatory, Integrative and Comparative Physiology*, 2000, vol. 279 (4), R1449-R1454
- **BENESCH et al.** How to Assess the Quality of Glucose Clamps? Evaluation of Clamps Performed With ClampArt, a Novel Automated Clamp Device. *Journal of Diabetes Science and Technology*, 2015, vol. 9 (4), 792-800
- **BERTHOUD, H.-R.** ; **NEUHUBER, W. L.** Functional and chemical anatomy of the afferent vagal system. *Autonomic Neuroscience*, 2000, vol. 85 (1-3), 1-17
- **BERTHOUD, H. R**. Anatomy and function of sensory hepatic nerves.. *Anat Rec A Discov Mol Cell Evol Biol*, 2004, vol. 280 (1), 827-835
- **BROM et al.** The effect of purification of Ga-68-labeled exendin on in vivo distribution.. *EJNMMI Res*, 2016, vol. 6 (1), 65
- **BURCELIN et al.** Glucose competence of the hepatoportal vein sensor requires the presence of an activated glucagon-like peptide-1 receptor.. *Diabetes*, 2001, vol. 50 (8), 1720-1728
- **CAMASTRA, S et al.** Early and longer term effects of gastric bypass surgery on tissue-specific insulin sensitivity and beta cell function in morbidly obese patients with and without type 2 diabetes.. *Diabetologia*, 2011, vol. 54 (8), 2093-2102
- **DADSON, P et al.** Effect of Bariatric Surgery on Adipose Tissue Glucose Metabolism in Different Depots in Patients With or Without Type 2 Diabetes. *Diabetes Care*, 2016, vol. 39 (2), 292-299
- **DANDONA, P. et al.** Mechanisms underlying the metabolic actions of testosterone in humans: A narrative review. *Diabetes Obes Metab*, 2021, vol. 23 (1), 18-28
- **EGNER, B.** High Definition Oscillometry: Non-invasive Blood Pressure Measurement and Pulse Wave Analysis.. *Handb Exp Pharmacol*, 2015, vol. 229, 243-264
- **ERIKSSON, O et al.** Glucagonlike Peptide-1 Receptor Imaging in Individuals with Type 2 Diabetes. *J Nucl Med*, 2022, vol. 63 (5), 794-800

- **ERIKSSON et al.** Species differences in pancreatic binding of DO3A-VS-Cys40 - Exendin4.. *Acta Diabetol*, 2017, vol. 54 (11), 1039-1045
- **FELBER, J.-P et al.** Glucose storage and oxidation in different degrees of human obesity measured by continuous indirect calorimetry. *Diabetologia*, 1981, vol. 20 (1), 39-44
- **GROSSMANN, M. et al.** Androgens and prostate cancer; pathogenesis and deprivation therapy.. *Best Pract Res Clin Endocrinol Metab*, 2013, vol. 27 (4), 603-616
- **HAIDER, K. S. et al.** Remission of type 2 diabetes following long-term treatment with injectable testosterone undecanoate in patients with hypogonadism and type 2 diabetes: 11-year data from a real-world registry study. *Diabetes Obes Metab*, 2020, vol. 22 (11), 2055-2068
- **HO, C. H et al.** Prediabetes is associated with an increased risk of testosterone deficiency, independent of obesity and metabolic syndrome.. *PLoS One*, 2013, vol. 8 (9), e74173
- **INTAGLIATA et al.** Diagnosis, Development, and Treatment of Portal Vein Thrombosis in Patients With and Without Cirrhosis.. *Gastroenterology*, 2019, vol. 156 (6), 1582-1599
- **IOZZO, P. et al.** Quantification of liver glucose metabolism by positron emission tomography: validation study in pigs.. *Gastroenterology*, 2007, vol. 132 (2), 531-542
- **IOZZO, P.** Metabolic imaging in obesity: underlying mechanisms and consequences in the whole body.. *Annals of the New York Academy of Sciences*, 2015, vol. 1353 (1), 21-40
- **IOZZO, P. et al.** 18F-FDG assessment of glucose disposal and production rates during fasting and insulin stimulation: a validation study. *Journal of nuclear medicine*, 2006, vol. 47 (6), 1016-1022
- **IZZI-ENGBEAYA et al.** Effects of Glucagon-like Peptide-1 on the Reproductive Axis in Healthy Men. *J Clin Endocrinol Metab*, 2020, vol. 105
- **JÉQUIER, E.** Energy metabolism in human obesity.. *Sozial-und Präventivmedizin*, 1989, vol. 34 (2), 58-62
- **JONES et al.** Targeting GLP-1 receptor trafficking to improve agonist efficacy. *Nat Commun*, 2018, vol. 9, 1602

- **KATO, A. et al.** Portal vein stent placement for the treatment of postoperative portal vein stenosis: long-term success and factor associated with stent failure.. *BMC Surg*, 2017, vol. 17 (1), 11
- **KODAMA, Y. et al.** Comparison of CT methods for determining the fat content of the liver.. *AJR Am J Roentgenol*, 2007, vol. 188 (5), 1307-1312
- **KOVACS, W. J. et al.** A mutation that causes lability of the androgen receptor under conditions that normally promote transformation to the DNA-binding state. *J Clin Invest*, 1984, vol. 73 (4), 1095-1104
- **KRIEGER et al.** Knockdown of GLP-1 Receptors in Vagal Afferents Affects Normal Food Intake and Glycemia.. *Diabetes*, 2016, vol. 65 (1), 34-43
- **MÄKINEN, J et al.** Obesity-associated intestinal insulin resistance is ameliorated after bariatric surgery.. *Diabetologia*, 2015, vol. 58 (5), 1055-1062
- **MALBERT, C. H et al.** Effects of chronic abdominal vagal stimulation of small-diameter neurons on brain metabolism and food intake.. *Brain Stimul*, 2017, vol. 10 (4), 735-743
- **MALBERT, C.-H et al.** Obesity-associated alterations in glucose metabolism are reversed by chronic bilateral stimulation of the abdominal vagus nerve.. *Diabetes*, 2017, vol. 66 (4), 848-857
- **MALBERT et al.** Pancreatic GLP-1r binding potential is reduced in insulin-resistant pigs. *BMJ Open Diabetes Res Care*, 2020, vol. 8 (2)
- **MALBERT et al.** Glucose-Sensing Mediated by Portal GLP-1 Receptor is Markedly Impaired in Insulin-Resistant Obese Animals.. *Diabetes.*, 2020
- **MALBERT, C.-H.** Open-source 3D printable frameless stereotaxic system for young and adult pigs. *Journal of Neuroscience Methods*, 2021, vol. 359, 109222
- **MALBERT, C.-H.** Vagally Mediated Gut-Brain Relationships in Appetite Control-Insights from Porcine Studies. *Nutrients*, 2021, vol. 13 (2), 467-484
- **MARZOOK et al.** The Interplay of Glucagon-Like Peptide-1 Receptor Trafficking and Signalling in Pancreatic Beta Cells. *Front Endocrinol (Lausanne)*, 2021, vol. 12, 678055
- **MITHIEUX, G.** *Détection du glucose sanguin par le système nerveux: pourquoi, où, comment.*, 2020
- **MIILLER et al.** Glucagon-like peptide 1 (GLP-1).. *Mol Metab*, 2019, vol. 30, 72-130
- **NAVARRO, G et al.** Extranuclear Actions of the Androgen Receptor Enhance Glucose-Stimulated Insulin Secretion in the Male. *Cell Metab*, 2016, vol. 23 (5), 837-851
- **NISHIZAWA et al.** Intraportal GLP-1 stimulates insulin secretion predominantly through the hepatoportal-pancreatic vagal reflex pathways. *Am J Physiol Endocrinol Metab*, 2013, vol. 305 (3), E376-87
- **PELTONIEMI, P et al.** Lumped constant for [(18)F]fluorodeoxyglucose in skeletal muscles of obese and nonobese humans.. *American Journal of Physiology-Endocrinology And Metabolism*, 2000, vol. 279 (5), E1122-30
- **PILLONETTO et al.** Dynamic insulin sensitivity index: importance in diabetes. *Am J Physiol Endocrinol Metab*, 2010, vol. 298 (3), E440-8
- **POULSEN, P. H. et al.** In vivo estimation of cerebral blood flow, oxygen consumption and glucose metabolism in the pig by [15O]water injection, [15O] oxygen inhalation and dual injections of [18F]fluorodeoxyglucose. *Journal of Neuroscience Methods*, 1997, vol. 77 (2), 199-209
- **REBELOS, E et al.** Insulin Resistance Is Associated With Enhanced Brain Glucose Uptake During Euglycemic Hyperinsulinemia: A Large-Scale PET Cohort.. *Diabetes Care*, 2021, vol. 44 (3), 788-794
- **SOLASS et al.** Morphology of the peritoneal cavity and pathophysiological consequences.. *Pleura and peritoneum*, 2016, vol. 1 (4), 193-201
- **SOTY et al.** Gut-Brain Glucose Signaling in Energy Homeostasis. *Cell Metab*, 2017, vol. 25 (6), 1231-1242
- **VAHL et al.** Glucagon-Like Peptide-1 (GLP-1) Receptors Expressed on Nerve Terminals in the Portal Vein Mediate the Effects of Endogenous GLP-1 on Glucose Tolerance in Rats.. *Endocrinology*, 2007, vol. 148 (10), 4965-4973
- **VAL-LAILLET et al.** A computed tomography scan application to evaluate adiposity in a minipig model of human obesity.. *Br J Nutr*, 2010, vol. 104 (11), 1719-1728
- **WU et al.** Development and Evaluation of 18F-TTCO-Cys40-Exendin-4: A PET Probe for Imaging Transplanted Islets. *Journal of Nuclear Medicine*, 2013, vol. 54 (2), 244-251
- **ZANOTTI-FREGONARA et al.** Image-derived input function for human brain using high resolution PET imaging with [C](R)-rolipram and [C]PBR28. *PLoS One*, 2011, vol. 6 (2), e17056
- **ZHU et al.** Glucagon-like peptide-1 receptor expression and its functions are regulated by androgen.. *Biomedicine & Pharmacotherapy*, 2019, vol. 120, 109555